# EUROPEAN PATENT APPLICATION

(11) **EP 4 549 589 A1**
(43) Date of publication of application: **07.05.2025**
(21) Application number: 23831581.6
(22) Date of filing: 29.06.2023
(51) Int. Cl.: C12Q 1/6895, A01H 6/14, A23L 33/105, C12N 15/11, C12P 15/00, C12Q 1/686

(54) **METHOD FOR SCREENING STEVIA PLANT**

(30) Priority: 30.06.2022 JP 2022106616
(71) Applicant: Suntory Holdings Limited, Osaka-shi, Osaka 530-8203 (JP)
(72) Inventor: HIRAI, Tadayoshi, Soraku-gun, Kyoto 619-0284 (JP); IWAKI, Kazunari, Kawasaki-shi, Kanagawa 211-0067 (JP); OCHIAI, Kentaro, Soraku-gun, Kyoto 619-0284 (JP); MIYAGAWA, Katsuro, Soraku-gun, Kyoto 619-0284 (JP); OKADA, Emi, Soraku-gun, Kyoto 619-0284 (JP)
(74) Representative: Mewburn Ellis LLP
(86) International application number: PCT/JP2023/024180
(87) International publication number: WO 2024/005142

(57) **Abstract**

Genetic information on stevia is elucidated. The present invention provides a method of screening for a stevia plant having at least one of the chemical features (a) to (r), comprising a step of detecting from the genome of a test stevia plant the presence and/or the absence of at least one of the genetic features (1) to (3), etc.

## Description

### [Technical Field]

The present invention relates to a method of screening for a stevia plant, etc.

### [Background Art]

In response to consumers' diversified needs, various drinks have been developed and are commercially available. Saccharides such as sucrose are components very commonly blended in drinks for the purpose of, for example, conferring sweetness. However, their influence on health due to excessive consumption has been pointed out. Thus, there are growing needs for lower calorie and naturally derived sweeteners. For example, Patent Literature 1 discloses a functional sweetener composition containing a vitamin, a high intensity sweetener, and a sweetness improving composition.

Steviol glycoside is known as a sweet component contained in a stevia extract. The stevia extract is mainly extracted and purified from a stevia leaf. Stevia is a perennial plant of the family Asteraceae with Paraguay in the South America as its place of origin, and its scientific name is Stevia rebaudiana Bertoni. Stevia contains a component having approximately 300 or more times the sweetness of sugar and is therefore cultivated for use of this sweet component extracted therefrom as a natural sweetener. The presence of various glycosides such as rebaudioside A (hereinafter, "rebaudioside " is also abbreviated as "Reb"), RebB, RebC, RebD, RebE and RebM has been reported as steviol glycoside (Patent Literature 2). Among various steviol glycosides, for example, RebA is evaluated as a high intensity sweetener having good quality of sweetness and is widely used. The other steviol glycosides have also been increasingly found to have their unique sweetness and associated taste.

Under these circumstances, a method of screening for a stevia plant characterized by the content of a steviol glycoside, etc. on the basis of genetic information is known (Patent Literature 3, Patent Literature 4, Patent Literature 5, and Patent Literature 6).

### [Citation List]

### [Patent Literature]

[Patent Literature 1] WO2007/070224
[Patent Literature 2] WO2010/038911
[Patent Literature 3] WO2016/049531
[Patent Literature 4] WO2019/074089
[Patent Literature 5] WO2021/230256
[Patent Literature 6] WO2021/230257

### [Disclosure of the Invention]

### [Technical Problem]

Further elucidation of genetic information on stevia is demanded.

### [Solution to Problem]

In one aspect, the present invention provides the following.
[1] A method of screening for a stevia plant having at least one of the following chemical features (a) to (r), comprising a step of detecting from the genome of a test stevia plant the presence and/or the absence of at least one of the following genetic features (1) to (3).
   (1) Heterozygous or homozygous for the allele wherein the base at the position corresponding to position 184 of SEQ ID NO: 1 is G.
   (2) Heterozygous or homozygous for the allele wherein the base at the position corresponding to position 170 of SEQ ID NO: 2 is C.
   (3) Heterozygous or homozygous for the allele wherein the base at the position corresponding to position 92 of SEQ ID NO: 3 is C.
      (a) The content ratio of rebaudioside (Reb) M to RebD is high as compared with a stevia plant that does not have any of the genetic features (1) to (3).
      (b) The content ratio of RebB to RebE is high as compared with a stevia plant that does not have any of the genetic features (1) to (3).
      (c) The content ratio of RebG to RebE is high as compared with a stevia plant that does not have any of the genetic features (1) to (3).
      (d) The content ratio of RebG to stevioside is high as compared with a stevia plant that does not have any of the genetic features (1) to (3).
      (e) The content of RebA is large as compared with a stevia plant that does not have any of the genetic features (1) to (3).
      (f) The content of RebB is large as compared with a stevia plant that does not have any of the genetic features (1) to (3).
      (g) The content of RebF is large as compared with a stevia plant that does not have any of the genetic features (1) to (3).
      (h) The content of RebG is large as compared with a stevia plant that does not have any of the genetic features (1) to (3).
      (i) The content of RebM is large as compared with a stevia plant that does not have any of the genetic features (1) to (3).
      (j) The content of a steviol glycoside having a β-1,3-glucoside bond is large as compared with a stevia plant that does not have any of the genetic features (1) to (3).
      (k) The content of RebD is small as compared with a stevia plant that does not have any of the genetic features (1) to (3).
      (l) The content of RebE is small as compared with a stevia plant that does not have any of the genetic features (1) to (3).
      (m) The content of RebN is small as compared with a stevia plant that does not have any of the genetic features (1) to (3).
      (n) The content of stevioside is small as compared with a stevia plant that does not have any of the genetic features (1) to (3).
      (o) The content of a steviol glycoside having no β-1,3-glucoside bond is small as compared with a stevia plant that does not have any of the genetic features (1) to (3).
      (p) The content ratio of any one of RebA, RebB, RebF, RebG, and RebM to any one of RebD, RebE, RebN, and stevioside is high as compared with a stevia plant that does not have any of the genetic features (1) to (3). The chemical feature (p) encompasses, for example, the embodiment of in which the content ratio of RebA to RebD is high and the content ratio of RebB toRebE is high.
      (q) The content ratio of a steviol glycoside having a β-1,3-glucoside bond to a steviol glycoside having no β-1,3-glucoside bond is high as compared with a stevia plant that does not have any of the genetic features (1) to (3).
      (r) The content ratio of a steviol glycoside produced by UGT76G1 from its substrate steviol glycoside to the substrate steviol glycoside of UGT76G1 is high as compared with a stevia plant that does not have any of the genetic features (1) to (3).
[2] The method according to [1], further comprising a step of measuring the content of RebA, RebB, RebD, RebE, RebF, RebG, RebM, RebN and/or stevioside in a test stevia plant tissue in which the presence and/or the absence of the genetic features has/have been detected.
[3] The method according to [1] or [2], wherein the step of detecting the presence and/or the absence of the genetic features is performed by use of CAPS method, dCAPS method or TaqMan PCR method.
[4] A screening kit for a stevia plant having at least one of the chemical features (a) to (r) defined in [1], comprising a reagent for detecting the presence and/or the absence of at least one of the genetic features (1) to (3) defined in [1].
[5] The kit according to [4], wherein the reagent comprises a primer and/or a probe for use in CAPS method, dCAPS method or TaqMan PCR method.
[6] A stevia plant having at least one of the genetic features (1) to (3) defined in [1].
[7] The plant according to [6], wherein the plant has at least one of the chemical features (a) to (r) defined in [1].
[8] The plant according to [6] or [7], wherein the plant is a non-genetically modified plant.
[9] The plant according to any one of [6] to [8], wherein the plant includes a stevia plant subjected to a mutagenesis treatment and a progeny plant thereof.
[10] A seed, a tissue, a dried leaf, a tissue culture or a cell of the plant according to any one of [6] to [9].
[11] The tissue, tissue culture or cell according to [10], which is selected from an embryo, a meristem cell, a pollen, a leaf, a root, a root apex, a petal, a protoplast, a leaf section and a callus.
[12] A method of producing a stevia plant having at least one of the chemical features (a) to (r) defined in [1], the method comprising a step of crossing the stevia plant according to any one of [6] to [9] with a second stevia plant.
[13] The method according to [12], wherein the second plant is the stevia plant according to any one of [6] to [9].
[14] A method of producing a stevia plant having at least one of the chemical features (a) to (r) defined in [1], comprising a step of modifying the genome of a stevia plant such that the genome has at least one of the genetic features (1) to (3) defined in [1].
[15] The method according to [14], wherein the modification of the genome is performed by a mutagenesis treatment.
[16] An extract of the plant according to any one of [6] to [9], or of the seed, tissue, dried leaf, tissue culture or cell according to [10] or [11], wherein the extract has at least one of the following chemical features (a') to (r').
   (a') The content ratio of rebaudioside (Reb) M to RebD is high as compared with an extract of a stevia plant that does not have any of the genetic features (1) to (3) defined in [1].
   (b') The content ratio of RebB to RebE is high as compared with an extract of a stevia plant that does not have any of the genetic features (1) to (3) defined in [1].
   (c') The content ratio of RebG to RebE is high as compared with an extract of a stevia plant that does not have any of the genetic features (1) to (3) defined in [1].
   (d') The content ratio of RebG to stevioside is high as compared with an extract of a stevia plant that does not have any of the genetic features (1) to (3) defined in [1].
   (e') The content of RebA is large as compared with an extract of a stevia plant that does not have any of the genetic features (1) to (3) defined in [1].
   (f') The content of RebB is large as compared with an extract of a stevia plant that does not have any of the genetic features (1) to (3) defined in [1].
   (g') The content of RebF is large as compared with an extract of a stevia plant that does not have any of the genetic features (1) to (3) defined in [1].
   (h') The content of RebG is large as compared with an extract of a stevia plant that does not have any of the genetic features (1) to (3) defined in [1].
   (i') The content of RebM is large as compared with an extract of a stevia plant that does not have any of the genetic features (1) to (3) defined in [1].
   (j') The content of a steviol glycoside having a β-1,3-glucoside bond is large as compared with an extract of a stevia plant that does not have any of the genetic features (1) to (3) defined in [1].
   (k') The content of RebD is small as compared with an extract of a stevia plant that does not have any of the genetic features (1) to (3) defined in [1].
   (l') The content of RebE is small as compared with an extract of a stevia plant that does not have any of the genetic features (1) to (3) defined in [1].
   (m') The content of RebN is small as compared with an extract of a stevia plant that does not have any of the genetic features (1) to (3) defined in [1].
   (n') The content of stevioside is small as compared with an extract of a stevia plant that does not have any of the genetic features (1) to (3) defined in [1].
   (o') The content of a steviol glycoside having no β-1,3-glucoside bond is small as compared with an extract of a stevia plant that does not have any of the genetic features (1) to (3) defined in [1].
   (p') The content ratio of any one of RebA, RebB, RebF, RebG, and RebM to any one of RebD, RebE, RebN, and stevioside is high as compared with an extract of a stevia plant that does not have any of the genetic features (1) to (3) defined in [1]. The chemical feature (p') encompasses, for example, the embodiment of in which the content ratio of RebA to RebD is high and the content ratio of RebB toRebE is high.
   (q') The content ratio of a steviol glycoside having a β-1,3-glucoside bond to a steviol glycoside having no β-1,3-glucoside bond is high as compared with an extract of a stevia plant that does not have any of the genetic features (1) to (3) defined in [1].
   (r') The content ratio of a steviol glycoside produced by UGT76G1 from its substrate steviol glycoside to the substrate steviol glycoside of UGT76G1 is high as compared with an extract of a stevia plant that does not have any of the genetic features (1) to (3) defined in [1].
[17] A method of producing an extract having at least one of the chemical features (a') to (r') defined in [16], comprising a step of obtaining an extract from the plant according to any one of [6] to [9], or from the seed, tissue, dried leaf, tissue culture or cell according to [10] or [11].
[18] A method of producing a steviol glycoside having a β-1,3-glucoside bond, comprising a step of purifying the steviol glycoside having a β-1,3-glucoside bond from the extract according to [16].
[19] A method of producing a food, a sweetener composition, a flavor or a medicament, comprising:
   a step of providing an extract of the plant according to any one of [6] to [9], an extract of the seed, tissue, dried leaf, tissue culture or cell according to [10] or [11], or the extract according to [16]; and
   a step of adding the extract to a raw material for the food, sweetener composition, flavor or medicament.
[20] The method according to [1], wherein the genetic feature (1) is heterozygous for the allele wherein the base at the position corresponding to position 184 of SEQ ID NO: 1 is G.
[21] The method according to [1], wherein the genetic feature (2) is heterozygous for the allele wherein the base at the position corresponding to position 170 of SEQ ID NO: 2 is C.
[22] The method according to [1], wherein the genetic feature (3) is homozygous for the allele wherein the base at the position corresponding to position 92 of SEQ ID NO: 3 is C.
   [10-2] A dead tissue or a dead cell of the plant according to any one of [6] to [9].
   [11-2] The dead tissue or dead cell according to [10-2], which is selected from an embryo, a meristem cell, a pollen, a leaf, a root, a root apex, a petal, a protoplast, a leaf section and a callus.
   [16-2] An extract of the dead tissue or dead cell according to [10-2] or [11-2], wherein the extract has at least one of the following chemical features (a') to (r').
      (a') The content ratio of RebM to rebaudioside (Reb) D is high as compared with an extract of a dead tissue or a dead cell of a stevia plant that does not have any of the genetic features (1) to (3) defined in [1].
      (b') The content ratio of RebB to RebE is high as compared with an extract of a dead tissue or a dead cell of a stevia plant that does not have any of the genetic features (1) to (3) defined in [1].
      (c') The content ratio of RebG to RebE is high as compared with an extract of a dead tissue or a dead cell of a stevia plant that does not have any of the genetic features (1) to (3) defined in [1].
      (d') The content ratio of RebG to stevioside is high as compared with an extract of a dead tissue or a dead cell of a stevia plant that does not have any of the genetic features (1) to (3) defined in [1].
      (e') The content of RebA is large as compared with an extract of a dead tissue or a dead cell of a stevia plant that does not have any of the genetic features (1) to (3) defined in [1].
      (f') The content of RebB is large as compared with an extract of a dead tissue or a dead cell of a stevia plant that does not have any of the genetic features (1) to (3) defined in [1].
      (g') The content of RebF is large as compared with an extract of a dead tissue or a dead cell of a stevia plant that does not have any of the genetic features (1) to (3) defined in [1].
      (h') The content of RebG is large as compared with an extract of a dead tissue or a dead cell of a stevia plant that does not have any of the genetic features (1) to (3) defined in [1].
      (i') The content of RebM is large as compared with an extract of a dead tissue or a dead cell of a stevia plant that does not have any of the genetic features (1) to (3) defined in [1].
      (j') The content of a steviol glycoside having a β-1,3-glucoside bond is large as compared with an extract of a dead tissue or a dead cell of a stevia plant that does not have any of the genetic features (1) to (3) defined in [1].
      (k') The content of RebD is small as compared with an extract of a dead tissue or a dead cell of a stevia plant that does not have any of the genetic features (1) to (3) defined in [1].
      (l') The content of RebE is small as compared with an extract of a dead tissue or a dead cell of a stevia plant that does not have any of the genetic features (1) to (3) defined in [1].
      (m') The content of RebN is small as compared with an extract of a dead tissue or a dead cell of a stevia plant that does not have any of the genetic features (1) to (3) defined in [1].
      (n') The content of stevioside is small as compared with an extract of a dead tissue or a dead cell of a stevia plant that does not have any of the genetic features (1) to (3) defined in [1].
      (o') The content of a steviol glycoside having no β-1,3-glucoside bond is small as compared with an extract of a dead tissue or a dead cell of a stevia plant that does not have any of the genetic features (1) to (3) defined in [1].
      (p') The content ratio of any one of RebA, RebB, RebF, RebG, and RebM to any one of RebD, RebE, RebN, and stevioside is high as compared with an extract of a dead tissue or a dead cell of a stevia plant that does not have any of the genetic features (1) to (3) defined in [1].
      (q') The content ratio of a steviol glycoside having a β-1,3-glucoside bond to a steviol glycoside having no β-1,3-glucoside bond is high as compared with an extract of a dead tissue or a dead cell of a stevia plant that does not have any of the genetic features (1) to (3) defined in [1].
      (r') The content ratio of a steviol glycoside produced by UGT76G1 from its substrate steviol glycoside to the substrate steviol glycoside of UGT76G1 is high as compared with an extract of a dead tissue or a dead cell of a stevia plant that does not have any of the genetic features (1) to (3) defined in [1].
   [17-2] A method of producing an extract having at least one of the chemical features (a') to (r') defined in [16-2], comprising a step of obtaining an extract from the dead tissue or dead cell according to [10-2] or [11-2].
   [18-2] A method of producing a steviol glycoside having a β-1,3-glucoside bond, comprising a step of purifying the steviol glycoside having a β-1,3-glucoside bond from the extract according to [16-2].
   [19-2] A method of producing a food, a sweetener composition, a flavor or a medicament, comprising:
      a step of providing an extract of the dead tissue or dead cell according to [10-2] or [11-2] or the extract according to [16-2]; and
      a step of adding the extract to a raw material for the food, sweetener composition, flavor or medicament.

### [Advantageous Effects of Invention]

The present invention enables the obtainment of a stevia plant having various characteristics described herein such as higher content ratio of RebM to RebD and the provision of an approach for producing such a plant, a leaf obtainable from such a plant, and a drink, etc. containing a steviol glycoside obtained from this leaf.

### [Brief Description of Drawings]

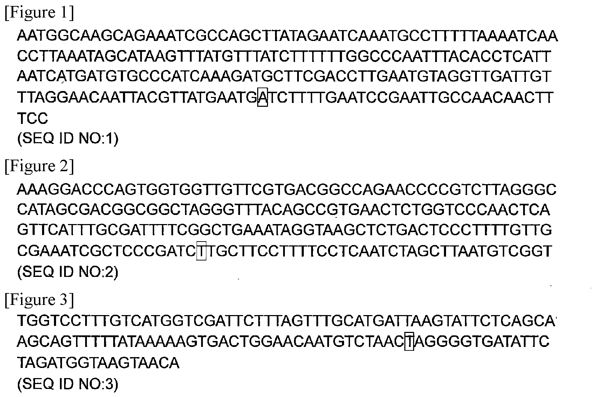

[Figure 1] Figure 1 is a diagram showing the position of the base related to the genetic feature (1) in the nucleotide sequence of SEQ ID NO: 1. The boxed base is a base related to the genetic feature (1).
[Figure 2] Figure 2 is a diagram showing the position of the base related to the genetic feature (2) in the nucleotide sequence of SEQ ID NO: 2. The boxed base is a base related to the genetic feature (2).
[Figure 3] Figure 3 is a diagram showing the position of the base related to the genetic feature (3) in the nucleotide sequence of SEQ ID NO: 3. The boxed base is a base related to the genetic feature (3).

### [Description of Embodiments]

Hereinafter, the present invention will be described in detail. The embodiments are given below merely for illustrating the present invention and are not intended to limit the present invention by such embodiments. The present invention can be carried out in various modes without departing from the spirit of the present invention.

Note that all documents, as well as laid-open application publications, patent application publications, and other patent documents cited herein shall be incorporated herein by reference.

### 1. Stevia plant

The present invention provides a stevia plant having at least one of the following genetic features (1) to (3) (hereinafter, generically referred to as the "plant of the present invention" or "stevia plant of the present invention").
(1) Heterozygous or homozygous, preferably heterozygous for the allele wherein the base at the position corresponding to position 184 of SEQ ID NO: 1 is G.
(2) Heterozygous or homozygous, preferably heterozygous for the allele wherein the base at the position corresponding to position 170 of SEQ ID NO: 2 is C.
(3) Heterozygous or homozygous, preferably homozygous for the allele wherein the base at the position corresponding to position 92 of SEQ ID NO: 3 is C.

The plant of the present invention is derived from a stevia plant of wild species and has at least one of the genetic features (1) to (3) (hereinafter, at least one of the genetic features (1) to (3) is generically referred to as the "genetic feature of the present invention").

The plant of the present invention can have at least one of the following chemical features.
(a) The content ratio of RebM to Reb D is high as compared with a stevia plant that does not have the genetic feature(s) of the present invention.
(b) The content ratio of RebB to RebE is high as compared with a stevia plant that does not have the genetic feature(s) of the present invention.
(c) The content ratio of RebG to RebE is high as compared with a stevia plant that does not have the genetic feature(s) of the present invention.
(d) The content ratio of RebG to stevioside is high as compared with a stevia plant that does not have the genetic feature(s) of the present invention.
(e) The content of RebA is large as compared with a stevia plant that does not have the genetic feature(s) of the present invention.
(f) The content of RebB is large as compared with a stevia plant that does not have the genetic feature(s) of the present invention.
(g) The content of RebF is large as compared with a stevia plant that does not have the genetic feature(s) of the present invention.
(h) The content of RebG is large as compared with a stevia plant that does not have the genetic feature(s) of the present invention.
(i) The content of RebM is large as compared with a stevia plant that does not have the genetic feature(s) of the present invention.
(j) The content of a steviol glycoside having a β-1,3-glucoside bond is large as compared with a stevia plant that does not have the genetic feature(s) of the present invention.
(k) The content of RebD is small as compared with a stevia plant that does not have the genetic feature(s) of the present invention.
(l) The content of RebE is small as compared with a stevia plant that does not have the genetic feature(s) of the present invention.
(m) The content of RebN is small as compared with a stevia plant that does not have the genetic feature(s) of the present invention.
(n) The content of stevioside is small as compared with a stevia plant that does not have the genetic feature(s) of the present invention.
(o) The content of a steviol glycoside having no β-1,3-glucoside bond is small as compared with a stevia plant that does not have the genetic feature(s) of the present invention.
(p) The content ratio of any one of RebA, RebB, RebF, RebG, and RebM to any one of RebD, RebE, RebN, and stevioside is high as compared with a stevia plant that does not have the genetic feature(s) of the present invention.
(q) The content ratio of a steviol glycoside having a β-1,3-glucoside bond to a steviol glycoside having no β-1,3-glucoside bond is high as compared with a stevia plant that does not have the genetic feature(s) of the present invention.
(r) The content ratio of a steviol glycoside produced by UGT76G1 from its substrate steviol glycoside to the substrate steviol glycoside of UGT76G1 is high as compared with a stevia plant that does not have the genetic feature(s) of the present invention.

The phrase "position (or portion) corresponding to" means the following. In case a sequence identical to a reference sequence (e.g., SEQ ID NOs: 1 to 3, etc.) is present in the genome, it means a position or a portion in the sequence (e.g., 184, 170, 92, etc.) present in the genome, and in case a sequence identical to the reference sequence is not present in the genome, it means a position or portion in a sequence corresponding to the reference sequence in the genome, which corresponds to the position or portion in the reference sequence. Whether or not a sequence identical to or corresponding to the reference sequence exists in the genome can be determined by, for example, amplifying genomic DNA of the stevia plant of interest with a primer capable of amplifying the reference sequence by PCR, sequencing the amplified product, and performing alignment analysis between the obtained sequence and the reference sequence. Non-limiting examples of a sequence corresponding to a reference sequence include, for example, a nucleotide sequence having a sequence identity of 60% or more, 70% or more, 75% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.1% or more, 98.4% or more, 98.7% or more, 99% or more, 99.2% or more, 99.5% or more, or 99.8% or more to the reference sequence. The position or portion corresponding to the position or portion in the reference sequence in the sequence corresponding to the reference sequence in the genome can be determined by taking into account the nucleotide sequence before and after the position or portion in the reference sequence and the like. For example, a position or portion in the sequence corresponding to the reference sequence in the genome, which corresponds to a position or portion in the reference sequence can be determined by an alignment analysis of a reference sequence with a sequence corresponding to a reference sequence in the genome.

For instance, when taking "the position corresponding to position 184 of SEQ ID NO: 1" of the genetic feature (1) of the present invention as an example, in case the genome of a stevia plant has a portion consisting of a nucleotide sequence identical to SEQ ID NO: 1, "the position corresponding to position 184 of SEQ ID NO: 1" is position 184 from the 5' end of the portion consisting of a nucleotide sequence identical to SEQ ID NO: 1 in the genome. On the other hand, in case the genome of a stevia plant has a portion consisting of a nucleotide sequence which is not identical to, but which corresponds to SEQ ID NO: 1, the genome does not have a portion consisting of a nucleotide sequence identical to SEQ ID NO: 1.
Therefore, "the position corresponding to position 184 of SEQ ID NO: 1" does not necessarily correspond to position 184 from the 5' end of the portion corresponding to SEQ ID NO: 1. However, it is possible to identify "the position corresponding to position 184 of SEQ ID NO: 1" in the genome of such a stevia plant by taking into account the nucleotide sequence before and after the position 184 of SEQ ID NO: 1, and the like. For instance, one can identify "the position corresponding to position 184 of SEQ ID NO: 1" in the genome of a stevia plant by an alignment analysis of the nucleotide sequence of a portion corresponding to SEQ ID NO: 1 in the genome of a stevia plant and the nucleotide sequence of SEQ ID NO: 1.

"The portion consisting of a nucleotide sequence corresponding to SEQ ID NO: 1" means, for instance, a portion consisting of a nucleotide sequence having a sequence identity of 60% or more, 70% or more, 75% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.1% or more, 98.4% or more, 98.7% or more, 99% or more, 99.2% or more, 99.5% or more, or 99.8% or more to the nucleotide sequence of SEQ ID NO: 1.

In some embodiments, "the portion consisting of a nucleotide sequence corresponding to SEQ ID NO: 1" includes a portion of the genome of a stevia plant which can be amplified by PCR using a forward primer (e.g., the one having the sequence of SEQ ID NO: 4) which hybridizes to a complementary sequence of a portion of positions 1 to 183 from the 5' end of SEQ ID NO: 1 (i.e., from the 5' end of SEQ ID NO: 1 to the base upstream by one base relative to the position 184 which relates to the genetic feature (1)) and a reverse primer (e.g., the one having the sequence of SEQ ID NO: 5) which hybridizes to a portion of positions 1 to 31 from the 3' end of SEQ ID NO: 1 (i.e., from the 3' end of SEQ ID NO: 1 to the base downstream by one base relative to the position 184 which relates to the genetic feature (1)).

In some embodiments, "the portion consisting of a nucleotide sequence corresponding to SEQ ID NO: 2" includes a portion of the genome of a stevia plant which can be amplified by PCR using a forward primer (e.g., the one having the sequence of SEQ ID NO: 6) which hybridizes to a complementary sequence of a portion of positions 1 to 169 from the 5' end of SEQ ID NO: 2 (i.e., from the 5' end of SEQ ID NO: 2 to the base upstream by one base relative to the position 170 which relates to the genetic feature (2)) and a reverse primer (e.g., the one having the sequence of SEQ ID NO: 7) which hybridizes to a portion of positions 1 to 34 from the 3' end of SEQ ID NO: 2 (i.e., from the 3' end of SEQ ID NO: 2 to the base downstream by one base relative to the position 170 which relates to the genetic feature (2)).

In some embodiments, "the portion consisting of a nucleotide sequence corresponding to SEQ ID NO: 3 " includes a portion of the genome of a stevia plant which can be amplified by PCR using a forward primer (e.g., the one having the sequence of SEQ ID NO: 8) which hybridizes to a complementary sequence of a portion of positions 1 to 91 from the 5' end of SEQ ID NO: 3 (i.e., from the 5' end of SEQ ID NO: 3 to the base upstream by one base relative to the position 92 which relates to the genetic feature (3)) and a reverse primer (e.g., the one having the sequence of SEQ ID NO: 9) which hybridizes to a portion of positions 1 to 29 from the 3' end of SEQ ID NO: 3 (i.e., from the 3' end of SEQ ID NO: 3 to the base downstream by one base relative to the position 92 which relates to the genetic feature (3)).

In a specific embodiment, "the portion consisting of a nucleotide sequence corresponding to SEQ ID NO: 1 " includes, for instance, a portion of the genome of a stevia plant which can be amplified by PCR using a forward primer comprising the nucleotide sequence of SEQ ID NO: 4 and a reverse primer comprising the nucleotide sequence of SEQ ID NO: 5.

In a specific embodiment, "the portion consisting of a nucleotide sequence corresponding to SEQ ID NO: 2" includes, for instance, a portion of the genome of a stevia plant which can be amplified by PCR using a forward primer comprising the nucleotide sequence of SEQ ID NO: 6 and a reverse primer comprising the nucleotide sequence of SEQ ID NO: 7.

In a specific embodiment, "the portion consisting of a nucleotide sequence corresponding to SEQ ID NO: 3 " includes, for instance, a portion of the genome of a stevia plant which can be amplified by PCR using a forward primer comprising the nucleotide sequence of SEQ ID NO: 8 and a reverse primer comprising the nucleotide sequence of SEQ ID NO: 9.

In a specific embodiment, "the allele wherein the base at the position corresponding to position 184 of SEQ ID NO: 1 is G" (hereinafter, may be referred to as "allele related to the genetic feature (1)") comprises the nucleotide sequence of at least one of SEQ ID NOs: 10 to 13.

In a specific embodiment, "the allele wherein the base at the position corresponding to position 170 of SEQ ID NO: 2 is C" (hereinafter, may be referred to as "allele related to the genetic feature (2)") comprises the nucleotide sequence of at least one of SEQ ID NOs: 14 to 17.

In a specific embodiment, "the allele wherein the base at the position corresponding to position 92 of SEQ ID NO: 3 is C" (hereinafter, may be referred to as "allele related to the genetic feature (3)") comprises the nucleotide sequence of at least one of SEQ ID NOs: 18 to 21.

Here, (1) the position corresponding to position 184 of SEQ ID NO: 1, (2) the position corresponding to position 170 of SEQ ID NO: 2, and (3) the position corresponding to position 92 of SEQ ID NO: 3, may be generically referred to as a "polymorphic site of the present invention" or a "site related to the genetic feature of the present invention".

The above genetic features can be detected by PCR method, TaqMan PCR method, sequencing method, microarray method, Invader method, TILLING method, RAD (random amplified polymorphic DNA) method, restriction fragment length polymorphism (RFLP) method, PCR-SSCP method, AFLP (amplified fragment length polymorphism) method, SSLP (simple sequence length polymorphism) method, CAPS (cleaved amplified polymorphic sequence) method, dCAPS (derived cleaved amplified polymorphic sequence) method, allele-specific oligonucleotide (ASO) method, ARMS method, denaturing gradient gel electrophoresis (DGGE) method, CCM (chemical cleavage of mismatch) method, DOL method, MALDI-TOF/MS method, TDI method, padlock probe method, molecular beacon method, DASH (dynamic allele specific hybridization) method, UCAN method, ECA method, PINPOINT method, PROBE (primer oligo base extension) method, VSET (very short extension) method, Survivor assay, Sniper assay, Luminex assay, GOOD method, LCx method, SNaPshot method, Mass ARRAY method, pyrosequencing method, SNP-IT method, melting curve analysis method, etc., but detection methods are not limited thereto.

In a specific embodiment, each genetic feature of the present invention is detectable by dCAPS method or the like using the following combination of a primer set and a restriction enzyme.

In case a candidate plant has the preferable genetic feature (1) (heterozygous), for example, a band of approximately 248 bp long (e.g., SEQ ID NO: 25), a band of approximately 213 bp long (e.g., SEQ ID NO: 26), and a band of approximately 35 bp long (e.g., SEQ ID NO: 27) are obtained by: performing PCR amplification using a forward primer having the nucleotide sequence shown in SEQ ID NO: 22 and a reverse primer having the nucleotide sequence shown in SEQ ID NO: 23 on the genomic DNA of the candidate plant; and treating the obtained PCR product (approximately 248 bp long, e.g., SEQ ID NO: 24 or 25) with a restriction enzyme EcoRV. On the other hand, in case the candidate plant does not have the genetic feature (1), a PCR product of approximately 248 bp long (e.g., SEQ ID NO: 24) is formed by performing PCR amplification in the same way as above, and when the PCR product is treated with the restriction enzyme, a band of approximately 213 bp long (e.g., SEQ ID NO: 26) and a band of approximately 35 bp long (e.g., SEQ ID NO: 27) are formed.

In case a candidate plant has the preferable genetic feature (2) (heterozygous), for example, a band of approximately 242 bp long (e.g., SEQ ID NO: 30), a band of approximately 207 bp long (e.g., SEQ ID NO: 32), and a band of approximately 35 bp long (e.g., SEQ ID NO: 33) are obtained by: performing PCR amplification using a forward primer having the nucleotide sequence shown in SEQ ID NO: 28 and a reverse primer having the nucleotide sequence shown in SEQ ID NO: 29 on the genomic DNA of the candidate plant; and treating the obtained PCR product (approximately 242 bp long, e.g., SEQ ID NO: 30 or 31) with a restriction enzyme PvuII. On the other hand, in case the candidate plant does not have the genetic feature (2), a PCR product of approximately 242 bp long (e.g., SEQ ID NO: 30) is formed by performing PCR amplification in the same way as above, and when the PCR product is treated with the restriction enzyme, only a band of an uncleaved PCR product of approximately 242 bp long (e.g., SEQ ID NO: 30) is obtained.

In case a candidate plant has the preferable genetic feature (3) (homozygous), for example, a band of approximately 249 bp long (e.g., SEQ ID NO: 37) and a band of approximately 35 bp long (e.g., SEQ ID NO: 38) are obtained by: performing PCR amplification using a forward primer having the nucleotide sequence shown in SEQ ID NO: 34 and a reverse primer having the nucleotide sequence shown in SEQ ID NO: 35 on the genomic DNA of the candidate plant; and treating the obtained PCR product (approximately 284 bp long, e.g., SEQ ID NO: 36) with a restriction enzyme PvuII. On the other hand, in case the candidate plant does not have the genetic feature (3), a PCR product of approximately 284 bp long (e.g., SEQ ID NO: 39) is formed by performing PCR amplification in the same way as above, and when the PCR product is treated with the restriction enzyme PvuII, only a band of an uncleaved PCR product of approximately 284 bp long (e.g., SEQ ID NO: 39) is obtained.

The term "approximately" as to bp long described above means ±5 bp. The restriction enzyme treatment can be performed according to conditions recommended by the distributor of each restriction enzyme used.

In some embodiments, the plant of the present invention has any one of the genetic features (1) to (3). In some embodiments, the plant of the present invention has a combination of the genetic features (1) and (2). In some embodiments, the plant of the present invention has a combination of the genetic features (1) and (3). In some embodiments, the plant of the present invention has a combination of the genetic features (2) and (3). In some embodiments, the plant of the present invention has all the genetic features (1) to (3).

The stevia plant of the present invention is derived from a stevia plant of wild species and has the above genetic features which result in at least one of the chemical features (a) to (r). The genetic features may be the ones generated by a genetic modification approach or the ones generated by a non-genetic modification approach. Therefore, the plant of the present invention may be the one obtained by a genetic modification approach or a progeny thereof (hereinafter, may be referred to as "genetically modified plant") or the one obtained by a non-genetic modification approach or a progeny thereof (hereinafter, may be referred to as "non-genetically modified plant").

Herein, examples of the "non-genetic modification approach" include a method of inducing a variation in the gene of a host cell (or a host plant) without transfection with a foreign gene. Examples of such a method include a method of allowing a mutagen to act on a plant cell. Examples of such a mutagen include ethylmethanesulfonic acid (EMS) and sodium azide. For example, EMS can be used at a concentration such as 0.1%, 0.2%, 0.3%, 0.4%, 0.5%, 0.6%, 0.7%, 0.8%, 0.9%, or 1.0% to treat a plant cell. The treatment time is about 1 hour to about 48 hours, about 2 hours to about 36 hours, about 3 hours to about 30 hours, about 4 hours to about 28 hours, about 5 hours to about 26 hours, or about 6 hours to about 24 hours. The procedures themselves of the treatment are known in the art and can be performed by dipping a water-absorbed seed obtained through a water absorption process in a treatment solution containing the mutagen at the concentration described above for the treatment time described above.

Another example of the non-genetic modification approach includes a method of irradiating a plant cell with radiation or light beam such as X-ray, γ ray, or ultraviolet ray. In the case of irradiation with ultraviolet ray, a cell irradiated using an appropriate dose (ultraviolet lamp intensity, distance, and time) of ultraviolet ray is cultured in a selective medium or the like, and then, a cell, a callus, or a plant having the trait of interest can be selected. In this operation, the irradiation intensity may be 0.01 to 100 Gr, 0.03 to 75 Gr, 0.05 to 50 Gr, 0.07 to 25 Gr, 0.09 to 20 Gr, 0.1 to 15 Gr, 0.1 to 10 Gr, 0.5 to 10 Gr, or 1 to 10 Gr. The irradiation distance may be 1 cm to 200 m, 5 cm to 100 m, 7 cm to 75 m, 9 cm to 50 m, 10 cm to 30 m, 10 cm to 20 m, or 10 cm to 10 m. The irradiation time may be 1 minute to 2 years, 2 minutes to 1 year, 3 minutes to 0.5 years, 4 minutes to 1 month, 5 minutes to 2 weeks, or 10 minutes to 1 week. The irradiation intensity, distance and time differ depending on the type of radiation or light beam, or the state of the subject to be irradiated (cell, callus, or plant) and can be appropriately adjusted by those skilled in the art.

Approaches such as cell fusion, another culture (haploid induction), and remote crossing (haploid induction) are also known in the art.

In general, plant cells may involve a mutation during culture. Therefore, it is preferred to regenerate a plant individual, for more stably maintaining the trait.

The scope of the present invention does not exclude a plant obtained by the ex-post facto genetic recombination (e.g., genome editing) with a non-genetically modified stevia plant as a host (e.g., a plant further provided with another trait by genetic recombination with the plant of the present invention as a host).

The plant of the present invention can have at least one of the chemical features (a) to (r) described above. In this context, the "stevia plant that does not have the genetic feature(s) of the present invention" means: for example, for a stevia plant having at least one of the genetic features (1) to (3) of the present invention, a stevia plant that does not have any of the genetic features (1) to (3) of the present invention; for a stevia plant having the genetic feature (1), a stevia plant other than the stevia plant having the genetic feature (1), i.e., a stevia plant that does not have the genetic feature (1); and for a stevia plant having the genetic features (1) and (2), a stevia plant other than the stevia plant having the genetic features (1) and (2), i.e., (i) a stevia plant that has neither the genetic feature (1) nor (2), (ii) a stevia plant that has the genetic feature (1), but does not have the genetic feature (2), and (iii) a stevia plant that has the genetic feature (2), but does not have the genetic feature (1).

The content ratio of a steviol glycoside to another steviol glycoside means the ratio of the content of the latter steviol glycoside (e.g., RebM) to the content of the former steviol glycoside (e.g., RebD) (e.g., the content of RebM / the content of RebD). The content may be, for example, the mass of the glycoside of interest contained in a unit mass of a stevia dried leaf or the concentration of the glycoside of interest in a stevia dried leaf. The dried leaf refers to a leaf having a water content decreased to 10% by weight or less, 7% by weight or less, 5% by weight or less, 4% by weight or less, 3% by weight or less, 2% by weight or less or 1% by weight or less by drying a fresh leaf. Preferably, the water content of the dried leaf of the plant of the present invention is 3 to 4% by weight.

In some embodiments, the content ratio of RebM to RebD in the plant of the present invention in relation to the chemical feature (a) is approximately 1.2 or more times, approximately 1.6 or more times, approximately 2 or more times, approximately 2.4 or more times, approximately 2.8 or more times, approximately 3.2 or more times, approximately 3.6 or more times, approximately 4 or more times, approximately 4.4 or more times, approximately 4.8 or more times, approximately 5.2 or more times, approximately 5.6 or more times, approximately 6 or more times, approximately 6.4 or more times, approximately 6.8 or more times, approximately 7.2 or more times, approximately 7.6 or more times, approximately 8 or more times, approximately 8.4 or more times, approximately 8.8 or more times, approximately 9.2 or more times, approximately 9.6 or more times, approximately 10 or more times, approximately 10.5 or more times, approximately 11 or more times, approximately 11.5 or more times, or approximately 12 or more times compared to the above ratio in a stevia plant that does not have the genetic feature(s) of the present invention.

In some embodiments, the content ratio of RebB to RebE in the plant of the present invention in relation to the chemical feature (b) is approximately 1.5 or more times, approximately 2 or more times, approximately 2.5 or more times, approximately 3 or more times, approximately 3.5 or more times, approximately 4 or more times, approximately 4.5 or more times, approximately 5 or more times, approximately 10 or more times, approximately 15 or more times, approximately 20 or more times, approximately 30 or more times, approximately 40 or more times, approximately 50 or more times, approximately 60 or more times, approximately 70 or more times, approximately 80 or more times, approximately 90 or more times, approximately 100 or more times, approximately 150 or more times, approximately 200 or more times, approximately 250 or more times, approximately 300 or more times, approximately 400 or more times, approximately 500 or more times, approximately 600 or more times, or approximately 700 or more times compared to the above ratio in a stevia plant that does not have the genetic feature(s) of the present invention.

In some embodiments, the content ratio of RebG to RebE in the plant of the present invention in relation to the chemical feature (c) is approximately 2 or more times, approximately 2.5 or more times, approximately 3 or more times, approximately 3.5 or more times, approximately 4 or more times, approximately 4.5 or more times, approximately 5 or more times, approximately 10 or more times, approximately 15 or more times, approximately 20 or more times, approximately 25 or more times, approximately 30 or more times, approximately 40 or more times, approximately 50 or more times, approximately 60 or more times, approximately 70 or more times, approximately 80 or more times, approximately 90 or more times, approximately 100 or more times, approximately 150 or more times, approximately 200 or more times, approximately 250 or more times, approximately 300 or more times, approximately 400 or more times, approximately 500 or more times, approximately 600 or more times, or approximately 700 or more times compared to the above ratio in a stevia plant that does not have the genetic feature(s) of the present invention.

In some embodiments, the content ratio of RebG to stevioside in the plant of the present invention in relation to the chemical feature (d) is approximately 1.5 or more times, approximately 2 or more times, approximately 2.5 or more times, approximately 3 or more times, approximately 3.5 or more times, approximately 4 or more times, approximately 4.5 or more times, approximately 5 or more times, approximately 10 or more times, approximately 15 or more times, approximately 20 or more times, approximately 30 or more times, approximately 40 or more times, approximately 50 or more times, approximately 60 or more times, approximately 70 or more times, approximately 80 or more times, approximately 90 or more times, approximately 100 or more times, approximately 150 or more times, approximately 200 or more times, approximately 250 or more times, approximately 300 or more times, approximately 400 or more times, approximately 500 or more times, approximately 600 or more times, or approximately 700 or more times compared to the above ratio in a stevia plant that does not have the genetic feature(s) of the present invention.

In some embodiments, the content of RebA in the plant of the present invention in relation to the chemical feature (e) is approximately 1.05 or more times, approximately 1.1 or more times, approximately 1.15 or more times, approximately 1.2 or more times, approximately 1.25 or more times, approximately 1.3 or more times, approximately 1.35 or more times, approximately 1.5 or more times, approximately 1.65 or more times, approximately 1.8 or more times, approximately 1.95 or more times, approximately 2.1 or more times, approximately 2.25 or more times, approximately 2.4 or more times, approximately 2.55 or more times, approximately 2.7 or more times, approximately 2.85 or more times, approximately 3 or more times, approximately 3.15 or more times, approximately 3.3 or more times, approximately 3.5 or more times, approximately 3.7 or more times, approximately 3.9 or more times, approximately 4.1 or more times, approximately 4.3 or more times, approximately 4.5 or more times, or approximately 4.7 or more times compared to the content of RebA in a stevia plant that does not have the genetic feature(s) of the present invention.

In some embodiments, the content of RebB in the plant of the present invention in relation to the chemical feature (f) is approximately 1.05 or more times, approximately 1.15 or more times, approximately 1.25 or more times, approximately 1.35 or more times, approximately 1.5 or more times, approximately 1.65 or more times, approximately 1.8 or more times, approximately 2 or more times, approximately 2.25 or more times, approximately 2.5 or more times, approximately 3 or more times, approximately 3.5 or more times, approximately 4 or more times, approximately 4.5 or more times, approximately 5 or more times, approximately 5.5 or more times, approximately 6 or more times, approximately 7 or more times, approximately 8 or more times, approximately 9 or more times, approximately 10 or more times, approximately 11 or more times, approximately 12 or more times, approximately 13 or more times, approximately 14 or more times, approximately 15 or more times, or approximately 16 or more times compared to the content of RebB in a stevia plant that does not have the genetic feature(s) of the present invention.

In some embodiments, the content of RebF in the plant of the present invention in relation to the chemical feature (g) is approximately 1.02 or more times, approximately 1.03 or more times, approximately 1.04 or more times, approximately 1.05 or more times, approximately 1.1 or more times, approximately 1.15 or more times, approximately 1.2 or more times, approximately 1.25 or more times, approximately 1.3 or more times, approximately 1.35 or more times, approximately 1.4 or more times, approximately 1.45 or more times, approximately 1.5 or more times, approximately 1.6 or more times, approximately 1.8 or more times, approximately 2 or more times, approximately 2.2 or more times, approximately 2.4 or more times, approximately 2.6 or more times, approximately 2.8 or more times, approximately 3 or more times, approximately 3.2 or more times, approximately 3.4 or more times, approximately 3.6 or more times, approximately 3.8 or more times, approximately 4 or more times, or approximately 4.2 or more times compared to the content of RebF in a stevia plant that does not have the genetic feature(s) of the present invention.

In some embodiments, the content of RebG in the plant of the present invention in relation to the chemical feature (h) is approximately 1.2 or more times, approximately 1.3 or more times, approximately 1.4 or more times, approximately 1.5 or more times, approximately 2 or more times, approximately 2.5 or more times, approximately 3 or more times, approximately 3.5 or more times, approximately 4 or more times, approximately 4.5 or more times, approximately 5 or more times, approximately 6 or more times, approximately 7 or more times, approximately 8 or more times, approximately 9 or more times, approximately 10 or more times, approximately 12.5 or more times, approximately 15 or more times, approximately 17.5 or more times, approximately 20 or more times, approximately 22.5 or more times, approximately 25 or more times, approximately 30 or more times, approximately 35 or more times, approximately 40 or more times, approximately 45 or more times, or approximately 50 or more times compared to the content of RebG in a stevia plant that does not have the genetic feature(s) of the present invention.

In some embodiments, the content of RebM in the plant of the present invention in relation to the chemical feature (i) is approximately 1.05 or more times, approximately 1.1 or more times, approximately 1.15 or more times, approximately 1.2 or more times, approximately 1.3 or more times, approximately 1.4 or more times, approximately 1.5 or more times, approximately 1.7 or more times, approximately 2 or more times, approximately 2.5 or more times, approximately 3 or more times, approximately 3.5 or more times, approximately 4 or more times, approximately 4.5 or more times, approximately 5 or more times, approximately 6 or more times, approximately 7 or more times, approximately 8 or more times, approximately 9 or more times, approximately 10 or more times, approximately 12 or more times, approximately 14 or more times, approximately 16 or more times, approximately 18 or more times, approximately 20 or more times, approximately 22 or more times, or approximately 24 or more times compared to the content of RebM in a stevia plant that does not have the genetic feature(s) of the present invention.

In some embodiments, the content of a steviol glycoside having a β-1,3-glucoside bond in the plant of the present invention in relation to the chemical feature (j) is approximately 1.02 or more times, approximately 1.03 or more times, approximately 1.04 or more times, approximately 1.05 or more times, approximately 1.1 or more times, approximately 1.15 or more times, approximately 1.2 or more times, approximately 1.3 or more times, approximately 1.4 or more times, approximately 1.5 or more times, approximately 2 or more times, approximately 2.5 or more times, approximately 3 or more times, approximately 3.5 or more times, approximately 4 or more times, approximately 4.5 or more times, approximately 5 or more times, approximately 6 or more times, approximately 7 or more times, approximately 8 or more times, approximately 9 or more times, approximately 10 or more times, approximately 12.5 or more times, approximately 15 or more times, approximately 17.5 or more times, approximately 20 or more times, approximately 22.5 or more times, approximately 25 or more times, approximately 30 or more times, approximately 35 or more times, approximately 40 or more times, approximately 45 or more times, or approximately 50 or more times compared to the content of the steviol glycoside having a β-1,3-glucoside bond in a stevia plant that does not have the genetic feature(s) of the present invention.

In some embodiments, the content of RebD in the plant of the present invention in relation to the chemical feature (k) is approximately 0.8 or less times, approximately 0.76 or less times, approximately 0.72 or less times, approximately 0.68 or less times, approximately 0.64 or less times, approximately 0.6 or less times, approximately 0.56 or less times, approximately 0.52 or less times, approximately 0.5 or less times, approximately 0.48 or less times, approximately 0.46 or less times, approximately 0.44 or less times, approximately 0.42 or less times, approximately 0.4 or less times, approximately 0.38 or less times, approximately 0.36 or less times, approximately 0.34 or less times, approximately 0.32 or less times, approximately 0.3 or less times, approximately 0.28 or less times, approximately 0.26 or less times, approximately 0.24 or less times, approximately 0.22 or less times, approximately 0.2 or less times, approximately 0.18 or less times, approximately 0.16 or less times, or approximately 0.14 or less times compared to the content of RebD in a stevia plant that does not have the genetic feature(s) of the present invention.

In some embodiments, the content of RebE in the plant of the present invention in relation to the chemical feature (l) is approximately 0.6 or less times, approximately 0.56 or less times, approximately 0.52 or less times, approximately 0.48 or less times, approximately 0.44 or less times, approximately 0.4 or less times, approximately 0.38 or less times, approximately 0.36 or less times, approximately 0.34 or less times, approximately 0.32 or less times, approximately 0.3 or less times, approximately 0.28 or less times, approximately 0.26 or less times, approximately 0.24 or less times, approximately 0.22 or less times, approximately 0.2 or less times, approximately 0.18 or less times, approximately 0.16 or less times, approximately 0.14 or less times, approximately 0.12 or less times, approximately 0.1 or less times, approximately 0.08 or less times, approximately 0.06 or less times, approximately 0.04 or less times, approximately 0.03 or less times, approximately 0.02 or less times, or approximately 0.01 or less times compared to the content of RebE in a stevia plant that does not have the genetic feature(s) of the present invention.

In some embodiments, the content of RebN in the plant of the present invention in relation to the chemical feature (m) is approximately 0.9 or less times, approximately 0.86 or less times, approximately 0.82 or less times, approximately 0.78 or less times, approximately 0.74 or less times, approximately 0.7 or less times, approximately 0.66 or less times, approximately 0.62 or less times, approximately 0.58 or less times, approximately 0.54 or less times, approximately 0.5 or less times, approximately 0.48 or less times, approximately 0.46 or less times, approximately 0.44 or less times, approximately 0.42 or less times, approximately 0.4 or less times, approximately 0.38 or less times, approximately 0.36 or less times, approximately 0.34 or less times, approximately 0.32 or less times, approximately 0.3 or less times, approximately 0.28 or less times, approximately 0.26 or less times, approximately 0.24 or less times, approximately 0.22 or less times, approximately 0.2 or less times, or approximately 0.18 or less times compared to the content of RebN in a stevia plant that does not have the genetic feature(s) of the present invention.

In some embodiments, the content of stevioside in the plant of the present invention in relation to the chemical feature (n) is approximately 0.8 or less times, approximately 0.75 or less times, approximately 0.7 or less times, approximately 0.65 or less times, approximately 0.6 or less times, approximately 0.55 or less times, approximately 0.5 or less times, approximately 0.45 or less times, approximately 0.4 or less times, approximately 0.35 or less times, approximately 0.3 or less times, approximately 0.28 or less times, approximately 0.26 or less times, approximately 0.24 or less times, approximately 0.22 or less times, approximately 0.2 or less times, approximately 0.18 or less times, approximately 0.16 or less times, approximately 0.14 or less times, approximately 0.12 or less times, approximately 0.1 or less times, approximately 0.08 or less times, approximately 0.06 or less times, approximately 0.05 or less times, approximately 0.04 or less times, approximately 0.03 or less times, or approximately 0.02 or less times compared to the content of stevioside in a stevia plant that does not have the genetic feature(s) of the present invention.

In some embodiments, the content of a steviol glycoside having no β-1,3-glucoside bond in the plant of the present invention in relation to the chemical feature (o) is approximately 0.9 or less times, approximately 0.86 or less times, approximately 0.82 or less times, approximately 0.78 or less times, approximately 0.74 or less times, approximately 0.7 or less times, approximately 0.66 or less times, approximately 0.62 or less times, approximately 0.6 or less times, approximately 0.56 or less times, approximately 0.52 or less times, approximately 0.48 or less times, approximately 0.44 or less times, approximately 0.4 or less times, approximately 0.38 or less times, approximately 0.36 or less times, approximately 0.34 or less times, approximately 0.32 or less times, approximately 0.3 or less times, approximately 0.28 or less times, approximately 0.26 or less times, approximately 0.24 or less times, approximately 0.22 or less times, approximately 0.2 or less times, approximately 0.18 or less times, approximately 0.16 or less times, approximately 0.14 or less times, approximately 0.12 or less times, approximately 0.1 or less times, approximately 0.08 or less times, approximately 0.06 or less times, approximately 0.04 or less times, approximately 0.03 or less times, approximately 0.02 or less times, or approximately 0.01 or less times compared to the content of the steviol glycoside having no β-1,3-glucoside bond in a stevia plant that does not have the genetic feature(s) of the present invention.

In some embodiments, the content ratio of any one of RebA, RebB, RebF, RebG, and RebM to any one of RebD, RebE, RebN, and stevioside in the plant of the present invention in relation to the chemical feature (p) is approximately 1.2 or more times, approximately 1.5 or more times, approximately 2 or more times, approximately 2.5 or more times, approximately 3 or more times, approximately 3.5 or more times, approximately 4 or more times, approximately 4.5 or more times, approximately 5 or more times, approximately 10 or more times, approximately 15 or more times, approximately 20 or more times, approximately 30 or more times, approximately 40 or more times, approximately 50 or more times, approximately 60 or more times, approximately 70 or more times, approximately 80 or more times, approximately 90 or more times, approximately 100 or more times, approximately 150 or more times, approximately 200 or more times, approximately 250 or more times, approximately 300 or more times, approximately 400 or more times, approximately 500 or more times, approximately 600 or more times, or approximately 700 or more times compared to the above ratio in a stevia plant that does not have the genetic feature(s) of the present invention.

In some embodiments, the content ratio of a steviol glycoside having a β-1,3-glucoside bond to a steviol glycoside having no β-1,3-glucoside bond in the plant of the present invention in relation to the chemical feature (q) is approximately 1.2 or more times, approximately 1.5 or more times, approximately 2 or more times, approximately 2.5 or more times, approximately 3 or more times, approximately 3.5 or more times, approximately 4 or more times, approximately 4.5 or more times, approximately 5 or more times, approximately 10 or more times, approximately 15 or more times, approximately 20 or more times, approximately 30 or more times, approximately 40 or more times, approximately 50 or more times, approximately 60 or more times, approximately 70 or more times, approximately 80 or more times, approximately 90 or more times, approximately 100 or more times, approximately 150 or more times, approximately 200 or more times, approximately 250 or more times, approximately 300 or more times, approximately 400 or more times, approximately 500 or more times, approximately 600 or more times, or approximately 700 or more times compared to the above ratio in a stevia plant that does not have the genetic feature(s) of the present invention.

In some embodiments, the content ratio of a steviol glycoside produced by UGT76G1 from its substrate steviol glycoside to the substrate steviol glycoside of UGT76G1 in the plant of the present invention in relation to the chemical feature (r) is approximately 1.2 or more times, approximately 1.5 or more times, approximately 2 or more times, approximately 3 or more times, approximately 4 or more times, approximately 5 or more times, approximately 6 or more times, approximately 8 or more times, approximately 10 or more times, approximately 15 or more times, approximately 20 or more times, approximately 25 or more times, approximately 30 or more times, approximately 35 or more times, approximately 40 or more times, approximately 45 or more times, approximately 50 or more times, approximately 55 or more times, approximately 60 or more times, approximately 65 or more times, approximately 70 or more times, approximately 75 or more times, approximately 80 or more times, approximately 85 or more times, approximately 90 or more times, approximately 95 or more times, or approximately 100 or more times compared to the above ratio in a stevia plant that does not have the genetic feature(s) of the present invention.

In some embodiments, the content ratio of RebM to RebD in the plant of the present invention in relation to the chemical feature (a) is approximately 0.12 or more, approximately 0.16 or more, approximately 0.2 or more, approximately 0.24 or more, approximately 0.28 or more, approximately 0.32 or more, approximately 0.36 or more, approximately 0.4 or more, approximately 0.44 or more, approximately 0.48 or more, approximately 0.5 or more, approximately 0.525 or more, approximately 0.55 or more, approximately 0.575 or more, approximately 0.6 or more, approximately 0.625 or more, approximately 0.65 or more, approximately 0.675 or more, approximately 0.7 or more, approximately 0.725 or more, approximately 0.75 or more, approximately 0.775 or more, approximately 0.8 or more, approximately 0.825 or more, approximately 0.85 or more, approximately 0.875 or more, approximately 0.9 or more, approximately 0.925 or more, approximately 0.95 or more, approximately 0.975 or more, or approximately 1 or more.

In some embodiments, the content ratio of RebB to RebE in the plant of the present invention in relation to the chemical feature (b) is approximately 0.17 or more, approximately 0.5 or more, approximately 1 or more, approximately 1.5 or more, approximately 2 or more, approximately 2.5 or more, approximately 3 or more, approximately 3.5 or more, approximately 4 or more, approximately 4.5 or more, approximately 5 or more, approximately 5.5 or more, approximately 6 or more, approximately 6.5 or more, approximately 7 or more, approximately 7.5 or more, approximately 8 or more, approximately 8.5 or more, approximately 9 or more, approximately 9.5 or more, approximately 10.1 or more, approximately 10.5 or more, approximately 11 or more, approximately 11.5 or more, approximately 12 or more, approximately 12.5 or more, approximately 13 or more, approximately 13.5 or more, approximately 14 or more, approximately 14.5 or more, or approximately 15 or more.

In some embodiments, the content ratio of RebG to RebE in the plant of the present invention in relation to the chemical feature (c) is approximately 0.004 or more, approximately 0.05 or more, approximately 0.1 or more, approximately 0.2 or more, approximately 0.3 or more, approximately 0.4 or more, approximately 0.5 or more, approximately 0.6 or more, approximately 0.7 or more, approximately 0.8 or more, approximately 0.9 or more, approximately 1.01 or more, approximately 1.02 or more, approximately 1.03 or more, approximately 1.04 or more, approximately 1.05 or more, approximately 1.07 or more, approximately 1.1 or more, approximately 1.12 or more, approximately 1.13 or more, approximately 1.17 or more, approximately 1.2 or more, approximately 1.25 or more, approximately 1.3 or more, approximately 1.35 or more, approximately 1.37 or more, approximately 1.38 or more, approximately 1.4 or more, approximately 1.5 or more, approximately 1.6 or more, approximately 1.7 or more, approximately 1.72 or more, approximately 1.75 or more, approximately 1.8 or more, approximately 1.85 or more, approximately 1.9 or more, approximately 1.95 or more, or approximately 2 or more.

In some embodiments, the content ratio of RebG to stevioside in the plant of the present invention in relation to the chemical feature (d) is approximately 0.0007 or more, approximately 0.004 or more, approximately 0.006 or more, approximately 0.008 or more, approximately 0.01 or more, approximately 0.012 or more, approximately 0.014 or more, approximately 0.016 or more, approximately 0.018 or more, approximately 0.02 or more, approximately 0.025 or more, approximately 0.03 or more, approximately 0.035 or more, approximately 0.04 or more, approximately 0.045 or more, approximately 0.05 or more, approximately 0.055 or more, approximately 0.06 or more, approximately 0.065 or more, approximately 0.07 or more, approximately 0.075 or more, approximately 0.08 or more, approximately 0.085 or more, approximately 0.09 or more, approximately 0.091 or more, approximately 0.092 or more, approximately 0.093 or more, approximately 0.094 or more, approximately 0.095 or more, approximately 0.096 or more, approximately 0.097 or more, approximately 0.098 or more, approximately 0.099 or more, or approximately 0.1 or more.

In some embodiments, the content of RebA in the plant of the present invention in relation to the chemical feature (e) is approximately 2% by weight or more, approximately 2.15% by weight or more, approximately 2.3% by weight or more, approximately 2.45% by weight or more, approximately 2.6% by weight or more, approximately 2.75% by weight or more, approximately 2.9% by weight or more, approximately 3.05% by weight or more, approximately 3.2% by weight or more, approximately 3.35% by weight or more, approximately 3.5% by weight or more, approximately 3.65% by weight or more, approximately 3.8% by weight or more, approximately 4% by weight or more, approximately 4.2% by weight or more, approximately 4.4% by weight or more, approximately 4.6% by weight or more, approximately 4.8% by weight or more, approximately 5% by weight or more, approximately 5.25% by weight or more, approximately 5.5% by weight or more, approximately 5.75% by weight or more, approximately 6% by weight or more, approximately 6.25% by weight or more, approximately 6.5% by weight or more, approximately 6.75% by weight or more, or approximately 7% by weight or more in terms of a concentration in a dried leaf.

In some embodiments, the content of RebB in the plant of the present invention in relation to the chemical feature (f) is approximately 0.04% by weight or more, approximately 0.044% by weight or more, approximately 0.048% by weight or more, approximately 0.052% by weight or more, approximately 0.056% by weight or more, approximately 0.06% by weight or more, approximately 0.064% by weight or more, approximately 0.068% by weight or more, approximately 0.072% by weight or more, approximately 0.076% by weight or more, approximately 0.08% by weight or more, approximately 0.084% by weight or more, approximately 0.088% by weight or more, approximately 0.092% by weight or more, approximately 0.096% by weight or more, approximately 0.1% by weight or more, approximately 0.105% by weight or more, approximately 0.11% by weight or more, approximately 0.115% by weight or more, approximately 0.12% by weight or more, approximately 0.125% by weight or more, approximately 0.13% by weight or more, approximately 0.135% by weight or more, approximately 0.14% by weight or more, approximately 0.145% by weight or more, approximately 0.15% by weight or more, or approximately 0.16% by weight or more in terms of a concentration in a dried leaf.

In some embodiments, the content of RebF in the plant of the present invention in relation to the chemical feature (g) is approximately 0.06% by weight or more, approximately 0.064% by weight or more, approximately 0.068% by weight or more, approximately 0.072% by weight or more, approximately 0.076% by weight or more, approximately 0.08% by weight or more, approximately 0.084% by weight or more, approximately 0.088% by weight or more, approximately 0.092% by weight or more, approximately 0.096% by weight or more, approximately 0.1% by weight or more, approximately 0.105% by weight or more, approximately 0.11% by weight or more, approximately 0.115% by weight or more, approximately 0.12% by weight or more, approximately 0.125% by weight or more, approximately 0.13% by weight or more, approximately 0.135% by weight or more, approximately 0.14% by weight or more, approximately 0.145% by weight or more, approximately 0.15% by weight or more, approximately 0.155% by weight or more, approximately 0.16% by weight or more, approximately 0.17% by weight or more, approximately 0.18% by weight or more, approximately 0.19% by weight or more, or approximately 0.2% by weight or more in terms of a concentration in a dried leaf.

In some embodiments, the content of RebG in the plant of the present invention in relation to the chemical feature (h) is approximately 0.001% by weight or more, approximately 0.002% by weight or more, approximately 0.003% by weight or more, approximately 0.004% by weight or more, approximately 0.005% by weight or more, approximately 0.006% by weight or more, approximately 0.007% by weight or more, approximately 0.008% by weight or more, approximately 0.009% by weight or more, approximately 0.01% by weight or more, approximately 0.011% by weight or more, approximately 0.012% by weight or more, approximately 0.013% by weight or more, approximately 0.014% by weight or more, approximately 0.016% by weight or more, approximately 0.018% by weight or more, approximately 0.02% by weight or more, approximately 0.022% by weight or more, approximately 0.024% by weight or more, approximately 0.026% by weight or more, approximately 0.028% by weight or more, approximately 0.03% by weight or more, approximately 0.032% by weight or more, approximately 0.034% by weight or more, approximately 0.036% by weight or more, approximately 0.038% by weight or more, or approximately 0.04% by weight or more in terms of a concentration in a dried leaf.

In some embodiments, the content of RebM in the plant of the present invention in relation to the chemical feature (i) is approximately 0.15% by weight or more, approximately 0.17% by weight or more, approximately 0.19% by weight or more, approximately 0.21% by weight or more, approximately 0.23% by weight or more, approximately 0.25% by weight or more, approximately 0.27% by weight or more, approximately 0.29% by weight or more, approximately 0.31% by weight or more, approximately 0.33% by weight or more, approximately 0.35% by weight or more, approximately 0.37% by weight or more, approximately 0.39% by weight or more, approximately 0.41% by weight or more, approximately 0.43% by weight or more, approximately 0.45% by weight or more, approximately 0.47% by weight or more, approximately 0.49% by weight or more, approximately 0.51% by weight or more, approximately 0.54% by weight or more, approximately 0.57% by weight or more, approximately 0.6% by weight or more, approximately 0.63% by weight or more, approximately 0.66% by weight or more, approximately 0.69% by weight or more, approximately 0.72% by weight or more, or approximately 0.75% by weight or more in terms of a concentration in a dried leaf.

In some embodiments, the content of a steviol glycoside having a β-1,3-glucoside bond in the plant of the present invention in relation to the chemical feature (j) is approximately 0.001% by weight or more, approximately 0.002% by weight or more, approximately 0.005% by weight or more, approximately 0.01% by weight or more, approximately 0.02% by weight or more, approximately 0.04% by weight or more, approximately 0.06% by weight or more, approximately 0.08% by weight or more, approximately 0.1% by weight or more, approximately 0.15% by weight or more, approximately 0.2% by weight or more, approximately 0.3% by weight or more, approximately 0.4% by weight or more, approximately 0.5% by weight or more, approximately 0.6% by weight or more, approximately 0.7% by weight or more, approximately 0.8% by weight or more, approximately 1% by weight or more, approximately 1.5% by weight or more, approximately 2% by weight or more, approximately 2.5% by weight or more, approximately 3% by weight or more, approximately 3.5% by weight or more, approximately 4% by weight or more, approximately 5% by weight or more, approximately 6% by weight or more, or approximately 7% by weight or more in terms of a concentration in a dried leaf.

In some embodiments, the content of RebD in the plant of the present invention in relation to the chemical feature (k) is approximately 1.65% by weight or less, approximately 1.6% by weight or less, approximately 1.55% by weight or less, approximately 1.5% by weight or less, approximately 1.45% by weight or less, approximately 1.4% by weight or less, approximately 1.35% by weight or less, approximately 1.3% by weight or less, approximately 1.25% by weight or less, approximately 1.2% by weight or less, approximately 1.15% by weight or less, approximately 1.1% by weight or less, approximately 1.05% by weight or less, approximately 1% by weight or less, approximately 0.95% by weight or less, approximately 0.9% by weight or less, approximately 0.85% by weight or less, approximately 0.8% by weight or less, approximately 0.75% by weight or less, approximately 0.7% by weight or less, approximately 0.65% by weight or less, approximately 0.6% by weight or less, approximately 0.55% by weight or less, approximately 0.5% by weight or less, approximately 0.45% by weight or less, approximately 0.4% by weight or less, or approximately 0.35% by weight or less in terms of a concentration in a dried leaf.

In some embodiments, the content of RebE in the plant of the present invention in relation to the chemical feature (l) is approximately 0.34% by weight or less, approximately 0.32% by weight or less, approximately 0.3% by weight or less, approximately 0.28% by weight or less, approximately 0.26% by weight or less, approximately 0.24% by weight or less, approximately 0.22% by weight or less, approximately 0.2% by weight or less, approximately 0.18% by weight or less, approximately 0.16% by weight or less, approximately 0.14% by weight or less, approximately 0.12% by weight or less, approximately 0.1% by weight or less, approximately 0.08% by weight or less, approximately 0.06% by weight or less, approximately 0.05% by weight or less, approximately 0.04% by weight or less, approximately 0.03% by weight or less, approximately 0.02% by weight or less, approximately 0.015% by weight or less, approximately 0.01% by weight or less, approximately 0.009% by weight or less, approximately 0.008% by weight or less, approximately 0.007% by weight or less, approximately 0.006% by weight or less, approximately 0.005% by weight or less, or approximately 0.004% by weight or less in terms of a concentration in a dried leaf.

In some embodiments, the content of RebN in the plant of the present invention in relation to the chemical feature (m) is approximately 0.35% by weight or less, approximately 0.33% by weight or less, approximately 0.31% by weight or less, approximately 0.29% by weight or less, approximately 0.27% by weight or less, approximately 0.25% by weight or less, approximately 0.24% by weight or less, approximately 0.23% by weight or less, approximately 0.22% by weight or less, approximately 0.21% by weight or less, approximately 0.2% by weight or less, approximately 0.19% by weight or less, approximately 0.18% by weight or less, approximately 0.17% by weight or less, approximately 0.16% by weight or less, approximately 0.15% by weight or less, approximately 0.14% by weight or less, approximately 0.13% by weight or less, approximately 0.12% by weight or less, approximately 0.11% by weight or less, approximately 0.1% by weight or less, approximately 0.09% by weight or less, approximately 0.08% by weight or less, approximately 0.07% by weight or less, approximately 0.06% by weight or less, approximately 0.055% by weight or less, or approximately 0.05% by weight or less in terms of a concentration in a dried leaf.

In some embodiments, the content of stevioside in the plant of the present invention in relation to the chemical feature (n) is approximately 2.5% by weight or less, approximately 2.4% by weight or less, approximately 2.3% by weight or less, approximately 2.2% by weight or less, approximately 2.1% by weight or less, approximately 2% by weight or less, approximately 1.9% by weight or less, approximately 1.8% by weight or less, approximately 1.7% by weight or less, approximately 1.6% by weight or less, approximately 1.5% by weight or less, approximately 1.4% by weight or less, approximately 1.3% by weight or less, approximately 1.2% by weight or less, approximately 1.1% by weight or less, approximately 1% by weight or less, approximately 0.9% by weight or less, approximately 0.8% by weight or less, approximately 0.7% by weight or less, approximately 0.6% by weight or less, approximately 0.5% by weight or less, approximately 0.45% by weight or less, approximately 0.4% by weight or less, approximately 0.35% by weight or less, approximately 0.3% by weight or less, approximately 0.25% by weight or less, or approximately 0.2% by weight or less in terms of a concentration in a dried leaf.

In some embodiments, the content of a steviol glycoside having no β-1,3-glucoside bond in the plant of the present invention in relation to the chemical feature (o) is approximately 2.5% by weight or less, approximately 2.2% by weight or less, approximately 2% by weight or less, approximately 1.8% by weight or less, approximately 1.6% by weight or less, approximately 1.4% by weight or less, approximately 1.2% by weight or less, approximately 1% by weight or less, approximately 0.9% by weight or less, approximately 0.8% by weight or less, approximately 0.7% by weight or less, approximately 0.6% by weight or less, approximately 0.5% by weight or less, approximately 0.4% by weight or less, approximately 0.3% by weight or less, approximately 0.2% by weight or less, approximately 0.1% by weight or less, approximately 0.08% by weight or less, approximately 0.06% by weight or less, approximately 0.04% by weight or less, approximately 0.02% by weight or less, approximately 0.015% by weight or less, approximately 0.01% by weight or less, approximately 0.008% by weight or less, approximately 0.006% by weight or less, approximately 0.005% by weight or less, or approximately 0.004% by weight or less in terms of a concentration in a dried leaf.

In some embodiments, the content ratio of any one of RebA, RebB, RebF, RebG, and RebM to any one of RebD, RebE, RebN, and stevioside in the plant of the present invention in relation to the chemical feature (p) is approximately 0.0007 or more, approximately 0.004 or more, approximately 0.01 or more, approximately 0.02 or more, approximately 0.03 or more, approximately 0.05 or more, approximately 0.08 or more, approximately 0.091 or more, approximately 0.095 or more, approximately 0.1 or more, approximately 0.2 or more, approximately 0.3 or more, approximately 0.5 or more, approximately 0.8 or more, approximately 1 or more, approximately 1.05 or more, approximately 1.07 or more, approximately 1.1 or more, approximately 1.2 or more, approximately 1.5 or more, approximately 2 or more, approximately 3 or more, approximately 4 or more, approximately 5 or more, approximately 8 or more, approximately 10 or more, approximately 10.5 or more, approximately 11 or more, approximately 11.5 or more, approximately 12 or more, approximately 12.5 or more, approximately 13 or more, approximately 13.5 or more, approximately 14 or more, approximately 14.5 or more, or approximately 15 or more.

In some embodiments, the content ratio of a steviol glycoside having a β-1,3-glucoside bond to a steviol glycoside having no β-1,3-glucoside bond in the plant of the present invention in relation to the chemical feature (q) is approximately 0.0007 or more, approximately 0.004 or more, approximately 0.01 or more, approximately 0.02 or more, approximately 0.03 or more, approximately 0.05 or more, approximately 0.08 or more, approximately 0.091 or more, approximately 0.095 or more, approximately 0.1 or more, approximately 0.2 or more, approximately 0.3 or more, approximately 0.5 or more, approximately 0.8 or more, approximately 1 or more, approximately 1.05 or more, approximately 1.07 or more, approximately 1.1 or more, approximately 1.2 or more, approximately 1.5 or more, approximately 2 or more, approximately 3 or more, approximately 4 or more, approximately 5 or more, approximately 8 or more, approximately 10 or more, approximately 10.5 or more, approximately 11 or more, approximately 11.5 or more, approximately 12 or more, approximately 12.5 or more, approximately 13 or more, approximately 13.5 or more, approximately 14 or more, approximately 14.5 or more, or approximately 15 or more.

In some embodiments, the content ratio of a steviol glycoside produced by UGT76G1 from its substrate steviol glycoside to the substrate steviol glycoside of UGT76G1 in the plant of the present invention in relation to the chemical feature (r) is approximately 0.15 or more, approximately 0.3 or more, approximately 0.5 or more, approximately 0.7 or more, approximately 0.9 or more, approximately 1 or more, approximately 1.5 or more, approximately 2 or more, approximately 3 or more, approximately 5 or more, approximately 7 or more, approximately 10 or more, approximately 15 or more, approximately 16 or more, approximately 17 or more, approximately 20 or more, approximately 25 or more, approximately 30 or more, approximately 35 or more, approximately 40 or more, approximately 45 or more, approximately 50 or more, approximately 51 or more, approximately 55 or more, approximately 60 or more, approximately 65 or more, approximately 66 or more, approximately 70 or more, approximately 75 or more, or approximately 80 or more.

In some embodiments, the steviol glycoside having a β-1,3-glucoside bond excludes RebD and RebN. In some embodiments, the steviol glycoside having a β-1,3-glucoside bond has two β-1,3-glucoside bonds. In an alternative embodiment, the steviol glycoside having a β-1,3-glucoside bond has one β-1,3-glucoside bond. In a preferable embodiment, the steviol glycoside having a β-1,3-glucoside bond is selected from the group consisting of RebA, RebB, RebF, RebH, RebG, RebI, RebJ, RebM, RebO, RebQ and dulcoside B. In a specific embodiment, the steviol glycoside having a β-1,3-glucoside bond is selected from the group consisting of RebA, RebB, RebF, RebG and RebM.

In some embodiments, the steviol glycoside having no β-1,3-glucoside bond is selected from the group consisting of steviolmonoside, steviolbioside, rubusoside, stevioside, RebE and dulcoside A. In a specific embodiment, the steviol glycoside having no β-1,3-glucoside bond is selected from the group consisting of stevioside and RebE.

In some embodiments, the substrate steviol glycoside of UGT76G1 is selected from steviolmonoside, rubusoside, steviolbioside, stevioside, RebA, RebD, RebE and RebG. In this embodiment, the "steviol glycoside produced by UGT76G1 from its substrate steviol glycoside" is steviol-1,3-bioside for steviolmonoside, RebG for rubusoside, RebB for steviolbioside, RebA for stevioside, RebI for RebA, RebM for RebD, RebD for RebE, and RebQ for RebG.

Each steviol glycoside can be extracted in the state of a liquid extract by reacting a fresh leaf or a dried leaf of the plant of the present invention with a suitable solvent (an aqueous solvent such as water or an organic solvent such as an alcohol, ether or acetone). For the extraction conditions, etc., see a method described in Ohta et al., J. Appl. Glycosci., Vol. 57, No. 3, 199-209 (2010) or WO2010/038911, or a method described in Examples mentioned later.

Further, individual steviol glycosides, for example, a steviol glycoside having a β-1,3-glucoside bond, RebA, RebB, RebF, RebG, and RebM, can be purified from the liquid extract thus obtained by use of a method known in the art such as a gradient of ethyl acetate or any of other organic solvents:water, high performance liquid chromatography (HPLC), gas chromatography, time-of-flight mass spectrometry (TOF-MS), or ultra (high) performance liquid chromatography (UPLC).

The content of each steviol glycoside can be measured by a method described in Ohta et al., supra or WO2010/038911, or a method described in Examples mentioned later. Specifically, for instance, a fresh leaf can be sampled from the stevia plant of the present invention, followed by measurement by LC-MS/MS and the like.

The plant of the present invention may include not only the whole plant but a plant organ (e.g., a leaf, a petal, a stem, a root, and a seed), a plant tissue (e.g., epidermis, phloem, soft tissue, xylem, vascular bundle, palisade tissue, and spongy tissue), various forms of plant cells (e.g., suspended cultured cells), a protoplast, a leaf section, a callus, and the like. The leaf may be a dried leaf mentioned above.

The plant of the present invention may also include a tissue culture or a cultured plant cell. This is because the plant can be regenerated by culturing such a tissue culture or a cultured plant cell. Examples of a regenerable form of the plant of the present invention include, but are not limited to, embryos, meristem cells, pollens, leaves, roots, root apices, petals, protoplasts, leaf sections and calluses.

### 2. Method of producing plant of present invention

In an alternative embodiment, the present invention provides a method of producing a stevia plant having at least one of the chemical features (a) to (r), the method comprising a step of crossing the stevia plant of the present invention with a second stevia plant (hereinafter, may be referred to as the "production method of the present invention").

The stevia plant produced by the method can have the same phenotype and genetic features as those of the plant of the present invention. The detailed individual chemical features in the plant obtainable by the production method of the present invention are as described above about the plant of the present invention.

In the production method of the present invention, "crossing" means that the plant of the present invention (first generation (S1)) is crossed with a second plant (S1) to obtain a daughter plant thereof (plant produced by the production method of the present invention (second generation (S2)). The crossing method is preferably backcross. The "backcross" is an approach of further crossing a daughter plant (S2) generated between the plant of the present invention and the second plant, with the plant of the present invention (i.e., a plant having the genetic feature(s) of the present invention) (S1) to produce a plant having the genetic feature(s) of the present invention. When the second plant (S1) for use in the production method of the present invention has the same phenotype and genetic features as those of the plant of the present invention, the crossing is substantially backcross.

Alternatively, the plant of the present invention can also be produced by selfing. The selfing can be performed by the self-pollination of the stamen pollen of the plant of the present invention with the pistil of the plant of the present invention.

When the plant produced by the production method of the present invention has the same phenotype and genetic features as those of the plant of the present invention, the plant produced by the production method of the present invention can be further crossed with a third stevia plant to produce a stevia plant having a phenotype equivalent to that of the plant of the present invention.

In an alternative embodiment, the plant of the present invention may be produced by regenerating a plant by the culture of the tissue culture or the cultured plant cell mentioned above. The culture conditions are the same as those for culturing a tissue culture or a cultured plant cell of the wild type stevia plant and are known in the art (Protocols for in vitro cultures and secondary metabolite analysis of aromatic and medicinal plants, Method in molecular biology, vol. 1391, pp. 113-123).

In a further alternative embodiment, the plant of the present invention may be produced by modifying the genome of a stevia plant so that the stevia plant is allowed to acquire the genetic features of the present invention. The acquirement of the genetic features of the present invention may be performed by a genetic modification approach (e.g., genome editing) or may be performed by a non-genetic modification approach. Examples of the non-genetic modification approach include mutagenesis treatment such as treatment with a mutagen and treatment by irradiation with radiation or light beam described in the section relating to the plant of the present invention. Specifically, for example, the genetic features of the present invention can be provided: to an individual having the allele wherein the base at the position corresponding to position 184 of SEQ ID NO: 1 is not G (e.g., the allele wherein the base at this position is A), by the substitution of the base at this position with G; to an individual having the allele wherein the base at the position corresponding to position 170 of SEQ ID NO: 2 is not C (e.g., the allele wherein the base at this position is T), by the substitution of the base at this position with C; to an individual having the allele wherein the base at the position corresponding to position 92 of SEQ ID NO: 3 is not C (e.g., the allele wherein the base at this position is T), by the substitution of the base at this position with C.

### 3. Method of screening for plant of present invention

The plant of the present invention or the plant having the same phenotype and/or genetic feature as those of the plant of the present invention can be screened for by detecting the genetic feature(s) of the present invention from a tissue of this plant. In this context, "screening" means that the plant of the present invention is discriminated from the other plants and selected.

Thus, in an alternative embodiment, the present invention provides a method of screening for a stevia plant having at least one of the chemical features (a) to (r), comprising a step of detecting the presence and/or the absence of at least one of the genetic features (1) to (3) from the genome of a test plant (hereinafter, may be referred to as the "screening method of the present invention").

In some embodiments, the genetic feature(s) to be detected is any one of the genetic features (1) to (3). In some embodiments, the genetic feature(s) to be detected is the genetic features (1) and (2). In some embodiments, the genetic feature(s) to be detected is the genetic features (1) and (3). In some embodiments, the genetic feature(s) to be detected is the genetic features (2) and (3). In some embodiments, the genetic feature(s) to be detected is all the genetic features (1) to (3).

The screening method of the present invention may further comprise a step of selecting from among the test plants a plant in which the presence of the above at least one genetic feature is detected.

The presence of the genetic features of the present invention can be determined, for example, by detecting the presence of an allele selected from the group consisting of:
(I) an allele wherein the base at the position corresponding to position 184 of SEQ ID NO: 1 is G (e.g., an allele comprising the nucleotide sequence of SEQ ID NO: 10, 11, 12 or 13);
(II) an allele wherein the base at the position corresponding to position 170 of SEQ ID NO: 2 is C (e.g., an allele comprising the nucleotide sequence of SEQ ID NO: 14, 15, 16 or 17); and
(III) an allele wherein the base at the position corresponding to position 92 of SEQ ID NO: 3 is C (e.g., an allele comprising the nucleotide sequence of SEQ ID NO: 18, 19, 20 or 21), and/or
detecting the absence of
(i) an allele wherein the base at the position corresponding to position 92 of SEQ ID NO: 3 is T (e.g., an allele comprising the nucleotide sequence of SEQ ID NO: 3, 46, 47 or 48).

The absence of the genetic features of the present invention can be determined, for example, by detecting the absence of an allele selected from the group consisting of:
(I) an allele wherein the base at the position corresponding to position 184 of SEQ ID NO: 1 is G (e.g., an allele comprising the nucleotide sequence of SEQ ID NO: 10, 11, 12 or 13);
(II) an allele wherein the base at the position corresponding to position 170 of SEQ ID NO: 2 is C (e.g., an allele comprising the nucleotide sequence of SEQ ID NO: 14, 15, 16 or 17); and
(III) an allele wherein the base at the position corresponding to position 92 of SEQ ID NO: 3 is C (e.g., an allele comprising the nucleotide sequence of SEQ ID NO: 18, 19, 20 or 21), and/or
detecting the presence of
(i) an allele wherein the base at the position corresponding to position 92 of SEQ ID NO: 3 is T (e.g., an allele comprising the nucleotide sequence of SEQ ID NO: 3, 46, 47 or 48).

Specific examples of methods of detecting the genetic features of the present invention include, but not limited to, PCR method, TaqMan PCR method, sequencing method, microarray method, Invader method, TILLING method, RAD method, RFLP method, PCR-SSCP method, AFLP method, SSLP method, CAPS method, dCAPS method, ASO method, ARMS method, DGGE method, CCM method, DOL method, MALDI-TOF/MS method, TDI method, padlock probe method, molecular beacon method, DASH method, UCAN method, ECA method, PINPOINT method, PROBE method, VSET method, Survivor assay, Sniper assay, Luminex assay, GOOD method, LCx method, SNaPshot method, Mass ARRAY method, pyrosequencing method, SNP-IT method, melting curve analysis method, etc.

In one embodiment, the genetic feature (1) of the present invention can be detected, for example, by dCAPS method using the following primer set and a restriction enzyme.

### Primer set:

A primer set comprising a forward primer comprising a sequence (e.g., SEQ ID NO: 22) of any continuous sequence of 15 bases or more which is positioned at the positions 1 to 211 of SEQ ID NO: 49 or 50 and a reverse primer comprising a continuous sequence of 15 to 36-base long from the 3' end of SEQ ID NO: 23.

### Restriction enzyme: EcoRV.

In one embodiment, the genetic feature (2) of the present invention can be detected, for example, by dCAPS method using the following primer set and a restriction enzyme.

### Primer set:

A primer set comprising a forward primer comprising a sequence (e.g., SEQ ID NO: 28) of any continuous sequence of 15 bases or more which is positioned at the positions 1 to 204 of SEQ ID NO: 51 or 52 and a reverse primer comprising a continuous sequence of 15 to 36-base long from the 3' end of SEQ ID NO: 29.

### Restriction enzyme: PvuII.

In one embodiment, the genetic feature (3) of the present invention can be detected, for example, by dCAPS method using the following primer set and a restriction enzyme.

### Primer set:

A primer set comprising a forward primer comprising a sequence (e.g., SEQ ID NO: 34) of any continuous sequence of 15 bases or more which is positioned at the positions 1 to 246 of SEQ ID NO: 53 or 54 and a reverse primer comprising a continuous sequence of 15 to 36-base long from the 3' end of SEQ ID NO: 35.

### Restriction enzyme: PvuII.

The sequences of the primers can be optimized within a range that satisfies the conditions described above. For the optimization of primer design, see, for example, Sambrook and Russell, "Molecular Cloning: A Laboratory Manual" 3rd Edition (2001), Cold Spring Harbor Laboratory Press. Each of the primers may be 15 to 50-base long, 18 to 48-base long, 20 to 45-base long, 30 to 40-base long, or the like. The restriction enzyme for each primer set also includes other enzymes that recognize the same sequence and cleave the same site as in the above enzyme, or an isoschizomer of the above enzyme. It is also possible to design a primer set other than those described above on the basis of the genetic feature(s) of the present invention and select a restriction enzyme appropriate therefor.

In a specific embodiment, the genetic features (1) to (3) of the present invention can be detected, e.g., by dCAPS method using the primer set having the following sequence and restriction enzyme.

**[Table 1]**

| Table 1 Examples of combination of primer set and restriction enzyme for detecting genetic features (1) to (3) | | | |
|---|---|---|---|
| Genetic feature | Forward primer | Reverse primer | Restriction enzyme |
| (1) | SEQ ID NO: 22 | SEQ ID NO: 23 | EcoRV |
| (2) | SEQ ID NO: 28 | SEQ ID NO: 29 | PvuII |
| (3) | SEQ ID NO: 34 | SEQ ID NO: 35 | PvuII |

The screening methods of the present invention may further comprise a step of measuring the content of steviol glycoside (e.g., the content of RebA, RebB, RebD, RebE, RebF, RebG, RebM, RebN and/or stevioside) of a tissue (e.g., a leave) of the test stevia plant for which the genetic features of the present invention have been detected. The measurement of the content of steviol glycoside is as described in the section "1. Stevia plant". In this embodiment, the screening method of the present invention may be applied to daughter plants obtained by selecting individuals with a higher degree of at least one of the chemical features (a) to (r) from among the test stevia plants in which the genetic feature(s) of the present invention is/are detected, and crossing the selected individuals with another stevia plants. Thus, the screening method of the present invention may comprise one or more of the following steps.
(i) Detecting the genetic features of the present invention from the genome of a test stevia plant;
(ii) measuring the content of steviol glycoside of the test stevia plant tissue in which the genetic features of the present invention have been detected;
(iii) selecting an individual with a higher degree of at least one of the chemical features (a) to (r) from among the test stevia plants in which the genetic features of the present invention have been detected;
(iv) crossing the selected individual with a higher degree of at least one of the chemical features (a) to (r) with another stevia plant;
(v) detecting the genetic features of the present invention from the genome of daughter plants obtained by crossing;
(vi) measuring the content of steviol glycoside of the tissue of the daughter plants in which the genetic features of the present invention have been detected;
(vii) selecting individuals with a higher degree of at least one of the chemical features (a) to (r) from among the daughter plants in which the genetic features of the present invention are detected.

The individual with a higher degree of chemical features means an individual with a higher content ratio of the steviol glycoside of interest as to the chemical features (a) to (d), (p) and (r), an individual with a larger content of the steviol glycoside of interest as to the chemical features (e) to (j), and an individual with a smaller content of the steviol glycoside of interest as to the chemical features (k) to (o), respectively.

Individuals with a high degree of the chemical feature of choice may be, for example, in top 50%, in top 40%, in top 30%, in top 20%, in top 10%, in top 5%, in top 4%, in top 3%, in top 2%, or in top 1% of the test stevia plants in which the genetic features of the present invention have been detected, with respect to the height of degree of the chemical feature. Other stevia plants to be crossed may or may not contain the genetic feature(s) of the present invention. In the above embodiment, steps (iv) to (vii) can be repeated a plurality of times. In this way, stevia plants with a higher degree of the chemical feature can be screened.

In the screening method of the present invention, the test stevia plant may be a natural plant or a non-genetically modified plant. Non-genetically modified plants are as described in the section "1. Stevia plant".

In the screening method of the present invention, the test stevia plant may include a stevia plant subjected to a mutagenesis treatment and a progeny plant thereof. The mutagenesis treatment is as described in the section "1. Stevia plant", and includes treatment with a mutagen, treatment with radiation or irradiation with light, and the like.

The present invention also provides the primer sets described above and combinations thereof, for example, the primer sets described above in Table 1 and combinations thereof. The present invention further provides a primer set capable of amplifying a region having a nucleotide sequence selected from the group consisting of SEQ ID NOs: 1 to 3, 10, 14 and 18 by PCR, for example, a primer set with a forward primer comprising a nucleotide sequence of SEQ ID NO: 4, and a reverse primer comprising a nucleotide sequence of SEQ ID NO: 5; a primer set with a forward primer comprising a nucleotide sequence of SEQ ID NO: 6, and a reverse primer comprising a nucleotide sequence of SEQ ID NO: 7; a primer set with a forward primer comprising a nucleotide sequence of SEQ ID NO: 8, and a reverse primer comprising a nucleotide sequence of SEQ ID NO: 9; and combinations of these primer sets.

In addition, the present invention provides a probe capable of detecting the presence and/or absence of the genetic features of the present invention, which may be referred to as the "probe of the present invention" hereinafter. The probe of the present invention may have a structure suitable for various detection methods (e.g., real-time PCR method such as TaqMan PCR method and the like) for the presence and/or absence of the genetic feature(s) of the present invention. For example, the probe of the present invention may comprise a nucleotide sequence complementary to a portion of a genome comprising a site related to the genetic feature of the present invention. Non-limiting examples of such probes include those comprising a sequence complementary to a nucleotide sequence selected from SEQ ID NOs: 11 to 13, 15 to 17, 19 to 21 and 40 to 48. Of these sequences, SEQ ID NOs: 11 to 13, 15 to 17 and 19 to 21 are specific for alleles related to the genetic feature of the present invention, and SEQ ID NOs: 40 to 48 are specific for alleles which are not the alleles related to the genetic feature of the present invention.

Further, SEQ ID NOs: 11 to 13 are specific for allele related to the genetic feature (1) of the present invention, SEQ ID NOs: 15 to 17 are specific for allele related to the genetic feature (2) of the present invention, and SEQ ID NOs: 19 to 21 are specific for allele related to the genetic feature (3) of the present invention. SEQ ID NOs: 40 to 42 are specific for an allele which is not the allele related to the genetic feature (1) of the present invention, SEQ ID NOs: 43 to 45 are specific for an allele which is not the allele related to the genetic feature (2) of the present invention, and SEQ ID NOs: 46 to 48 are specific for an allele which is not the allele related to the genetic feature (3) of the present invention.

The presence of the genetic feature(s) of the present invention may be detected by detection of an allele related to the genetic feature of the present invention, and the absence of the genetic feature(s) of the invention may be detected by undetection of an allele related to the genetic feature of the present invention. The presence of the genetic feature (3) of the present invention may be detected by undetection of an allele which is not an allele related to the genetic feature (3) of the present invention, and the absence of the genetic feature (3) of the invention may be detected by detection of an allele which is not an allele related to the genetic feature (3) of the present invention.

The probes of the present invention preferably have a label. Non-limiting examples of such labels include fluorescent labels, luminescent labels, radioactive labels, dyes, enzymes, quenchers, binding moieties with detectable labels, and the like. In a specific embodiment, the probe of the present invention has a polynucleotide comprising a nucleotide sequence complementary to a sequence selected from SEQ ID NOs: 11 to 13, 15 to 17, 19 to 21 and 40 to 48 and a label.

The present invention also provides a kit comprising the above-mentioned primer set and a restriction enzyme appropriate therefor. In a specific embodiment, the kit of the present invention comprises a primer set comprising a combination of a forward primer and a reverse primer stated in the above Table 1 and a restriction enzyme appropriate therefor.

The present invention also provides a kit comprising a primer set capable of amplifying by PCR a region having a nucleotide sequence selected from the group consisting of SEQ ID NOs: 1 to 3, 10, 14 and 18, and the above-mentioned probe of the present invention appropriate therefor.

These primer sets, probes and kits can be used to detect the genetic feature(s) of the present invention, used in the screening methods of the present invention, and the like. These primer sets and kits may also comprise an instruction including an explanation on the detection of genetic feature(s) of the present invention and on the screening method of the present invention, e.g., a written instruction, information of a site comprising information regarding the method of use (e.g., URL and 2D code), and media (e.g., a flexible disk, a CD, a DVD, a Blu-ray disk, a memory card, a USB memory) etc., having recorded thereon information regarding the method of use, and the like.

In some embodiments, the present invention provides a screening kit for the stevia plant having at least one of the chemical features (a) to (r), comprising a reagent for detecting the presence and/or the absence of at least one of the genetic features (1) to (3). The reagent may comprise a primer and/or a probe for use in CAPS method, dCAPS method or TaqMan PCR method. In a specific embodiment, the reagent for detecting the presence and/or the absence of at least one of the genetic features (1) to (3) comprises a combination of a primer set and a restriction enzyme for detecting at least one of the above genetic features (1) to (3) by the dCAPS method, for instance a combination of a primer set and a restriction enzyme stated in Table 1 described above, or a combination of a primer set that amplifies the site(s) related to at least one of the genetic features (1) to (3) (e.g., a site comprising a sequence selected from SEQ ID NOs: 10 to 21), and a probe having a nucleotide sequence complementary to a site related to at least one of the genetic features (1) to (3) (e.g., a site comprising a sequence selected from SEQ ID NOs: 11 to 13, 15 to 17 and 19 to 21), which can be used in the TaqMan PCR method or the like.

### 4. Method of producing extract derived from plant and product comprising extract

In a further embodiment, the present invention provides a method of producing an extract having at least one of the following chemical features (a') to (r'), comprising a step of obtaining an extract from the plant of the present invention, a stevia plant selected by the screening method of the present invention or a stevia plant produced by the production method of the present invention, or a seed, a leaf (e.g., dried leaf or fresh leaf), a tissue, a tissue culture or a cell of the plant (hereinafter, may be referred to as the "extract production method of the present invention").
(a') The content ratio of RebM to RebD is high as compared with an extract of a stevia plant that does not have the genetic feature(s) of the present invention.
(b') The content ratio of RebB to RebE is high as compared with an extract of a stevia plant that does not have the genetic feature(s) of the present invention.
(c') The content ratio of RebG to RebE is high as compared with an extract of a stevia plant that does not have the genetic feature(s) of the present invention.
(d') The content ratio of RebG to stevioside is high as compared with an extract of a stevia plant that does not have the genetic feature(s) of the present invention.
(e') The content of RebA is large as compared with an extract of a stevia plant that does not have the genetic feature(s) of the present invention.
(f') The content of RebB is large as compared with an extract of a stevia plant that does not have the genetic feature(s) of the present invention.
(g') The content of RebF is large as compared with an extract of a stevia plant that does not have the genetic feature(s) of the present invention.
(h') The content of RebG is large as compared with an extract of a stevia plant that does not have the genetic feature(s) of the present invention.
(i') The content of RebM is large as compared with an extract of a stevia plant that does not have the genetic feature(s) of the present invention.
(j') The content of a steviol glycoside having a β-1,3-glucoside bond is large as compared with an extract of a stevia plant that does not have the genetic feature(s) of the present invention.
(k') The content of RebD is small as compared with an extract of a stevia plant that does not have the genetic feature(s) of the present invention.
(l') The content of RebE is small as compared with an extract of a stevia plant that does not have the genetic feature(s) of the present invention.
(m') The content of RebN is small as compared with an extract of a stevia plant that does not have the genetic feature(s) of the present invention.
(n') The content of stevioside is small as compared with an extract of a stevia plant that does not have the genetic feature(s) of the present invention.
(o') The content of a steviol glycoside having no β-1,3-glucoside bond is small as compared with an extract of a stevia plant that does not have the genetic feature(s) of the present invention.
(p') The content ratio of any one of RebA, RebB, RebF, RebG, and RebM to any one of RebD, RebE, RebN, and stevioside is high as compared with an extract of a stevia plant that does not have the genetic feature(s) of the present invention.
(q') The content ratio of a steviol glycoside having a β-1,3-glucoside bond to a steviol glycoside having no β-1,3-glucoside bond is high as compared with an extract of a stevia plant that does not have the genetic feature(s) of the present invention.
(r') The content ratio of a steviol glycoside produced by UGT76G1 from its substrate steviol glycoside to the substrate steviol glycoside of UGT76G1 is high as compared with an extract of a stevia plant that does not have the genetic feature(s) of the present invention.

Further provided is an extract having at least one of the chemical features (a') to (r') from the plant of the present invention, a stevia plant selected by the screening method of the present invention or a stevia plant produced by the production method of the present invention, or a seed, a leaf (e.g., dried leaf or fresh leaf), a tissue, a tissue culture or a cell of the plant (hereinafter, may be referred to as the "extract of the present invention"). The extract of the present invention is preferably produced by the extract production method of the present invention. The extract of the present invention may be unpurified (an unpurified extract or a crude extract) or may be purified (a purified extract). Furthermore, provided is a method of producing the steviol glycoside, comprising a step of purifying the steviol glycoside from the extract of the present invention (hereinafter, may be referred to as the "steviol glycoside production method of the present invention"). The steviol glycoside production method of the present invention may further comprise a step of obtaining an extract having at least one of the chemical features (a') to (r') from the plant of the present invention, a stevia plant selected by the screening method of the present invention or a stevia plant produced by the production method of the present invention. Examples of the steviol glycoside that is produced by the steviol glycoside production method of the present invention include, but are not particularly limited to, RebA, RebB, RebC, RebD, RebE, RebF, RebG, RebI, RebJ, RebM, RebN, RebO, RebQ, stevioside, steviolbioside, steviolmonoside, dulcoside A, dulcoside B, and rubusoside. In a preferable embodiment, the steviol glycoside is a steviol glycoside having a β-1,3-glucoside bond. In a more preferable embodiment, a content or content ratio of the steviol glycoside in the plant of the present invention is higher than in a stevia plant that does not have the genetic feature(s) of the present invention. In a particularly preferable embodiment, the steviol glycoside is selected from RebA, RebB, RebF, RebG, RebM and a combination thereof.

The extract having at least one of the chemical features (a') to (r') can be obtained by reacting a fresh leaf or a dried leaf of the plant of the present invention with a suitable solvent (an aqueous solvent such as water or an organic solvent such as an alcohol, ether or acetone). For the extraction conditions, etc., see a method described in Ohta et al., supra or WO2010/038911, or a method described in Examples mentioned later.

The steviol glycoside can be purified from the extract having at least one of the chemical features (a') to (r') by use of a method known in the art such as a gradient of ethyl acetate or any of other organic solvents:water, high performance liquid chromatography (HPLC), gas chromatography, time-of-flight mass spectrometry (TOF-MS), or ultra (high) performance liquid chromatography (UPLC).

In some embodiments, the content ratio of RebM to RebD in the extract of the present invention in relation to the chemical feature (a') is approximately 1.2 or more times, approximately 1.6 or more times, approximately 2 or more times, approximately 2.4 or more times, approximately 2.8 or more times, approximately 3.2 or more times, approximately 3.6 or more times, approximately 4 or more times, approximately 4.4 or more times, approximately 4.8 or more times, approximately 5.2 or more times, approximately 5.6 or more times, approximately 6 or more times, approximately 6.4 or more times, approximately 6.8 or more times, approximately 7.2 or more times, approximately 7.6 or more times, approximately 8 or more times, approximately 8.4 or more times, approximately 8.8 or more times, approximately 9.2 or more times, approximately 9.6 or more times, approximately 10 or more times, approximately 10.5 or more times, approximately 11 or more times, approximately 11.5 or more times, or approximately 12 or more times compared to the above ratio in an extract obtained by the same production method as that for the extract of the present invention from a stevia plant that does not have the genetic feature(s) of the present invention (hereinafter, referred to as a control extract).

In some embodiments, the content ratio of RebB to RebE in the extract of the present invention in relation to the chemical feature (b') is approximately 1.5 or more times, approximately 2 or more times, approximately 2.5 or more times, approximately 3 or more times, approximately 3.5 or more times, approximately 4 or more times, approximately 4.5 or more times, approximately 5 or more times, approximately 10 or more times, approximately 15 or more times, approximately 20 or more times, approximately 30 or more times, approximately 40 or more times, approximately 50 or more times, approximately 60 or more times, approximately 70 or more times, approximately 80 or more times, approximately 90 or more times, approximately 100 or more times, approximately 150 or more times, approximately 200 or more times, approximately 250 or more times, approximately 300 or more times, approximately 400 or more times, approximately 500 or more times, approximately 600 or more times, or approximately 700 or more times compared to the above ratio in a control extract.

In some embodiments, the content ratio of RebG to RebE in the extract of the present invention in relation to the chemical feature (c') is approximately 2 or more times, approximately 2.5 or more times, approximately 3 or more times, approximately 3.5 or more times, approximately 4 or more times, approximately 4.5 or more times, approximately 5 or more times, approximately 10 or more times, approximately 15 or more times, approximately 20 or more times, approximately 25 or more times, approximately 30 or more times, approximately 40 or more times, approximately 50 or more times, approximately 60 or more times, approximately 70 or more times, approximately 80 or more times, approximately 90 or more times, approximately 100 or more times, approximately 150 or more times, approximately 200 or more times, approximately 250 or more times, approximately 300 or more times, approximately 400 or more times, approximately 500 or more times, approximately 600 or more times, or approximately 700 or more times compared to the above ratio in a control extract.

In some embodiments, the content ratio of RebG to stevioside in the extract of the present invention in relation to the chemical feature (d') is approximately 1.5 or more times, approximately 2 or more times, approximately 2.5 or more times, approximately 3 or more times, approximately 3.5 or more times, approximately 4 or more times, approximately 4.5 or more times, approximately 5 or more times, approximately 10 or more times, approximately 15 or more times, approximately 20 or more times, approximately 30 or more times, approximately 40 or more times, approximately 50 or more times, approximately 60 or more times, approximately 70 or more times, approximately 80 or more times, approximately 90 or more times, approximately 100 or more times, approximately 150 or more times, approximately 200 or more times, approximately 250 or more times, approximately 300 or more times, approximately 400 or more times, approximately 500 or more times, approximately 600 or more times, or approximately 700 or more times compared to the above ratio in a control extract.

In some embodiments, the content of RebA in the extract of the present invention in relation to the chemical feature (e') is approximately 1.05 or more times, approximately 1.1 or more times, approximately 1.15 or more times, approximately 1.2 or more times, approximately 1.25 or more times, approximately 1.3 or more times, approximately 1.35 or more times, approximately 1.5 or more times, approximately 1.65 or more times, approximately 1.8 or more times, approximately 1.95 or more times, approximately 2.1 or more times, approximately 2.25 or more times, approximately 2.4 or more times, approximately 2.55 or more times, approximately 2.7 or more times, approximately 2.85 or more times, approximately 3 or more times, approximately 3.15 or more times, approximately 3.3 or more times, approximately 3.5 or more times, approximately 3.7 or more times, approximately 3.9 or more times, approximately 4.1 or more times, approximately 4.3 or more times, approximately 4.5 or more times, or approximately 4.7 or more times compared to the content of RebA in a control extract.

In some embodiments, the content of RebB in the extract of the present invention in relation to the chemical feature (f) is approximately 1.05 or more times, approximately 1.15 or more times, approximately 1.25 or more times, approximately 1.35 or more times, approximately 1.5 or more times, approximately 1.65 or more times, approximately 1.8 or more times, approximately 2 or more times, approximately 2.25 or more times, approximately 2.5 or more times, approximately 3 or more times, approximately 3.5 or more times, approximately 4 or more times, approximately 4.5 or more times, approximately 5 or more times, approximately 5.5 or more times, approximately 6 or more times, approximately 7 or more times, approximately 8 or more times, approximately 9 or more times, approximately 10 or more times, approximately 11 or more times, approximately 12 or more times, approximately 13 or more times, approximately 14 or more times, approximately 15 or more times, or approximately 16 or more times compared to the content of RebB in a control extract.

In some embodiments, the content of RebF in the extract of the present invention in relation to the chemical feature (g') is approximately 1.02 or more times, approximately 1.03 or more times, approximately 1.04 or more times, approximately 1.05 or more times, approximately 1.1 or more times, approximately 1.15 or more times, approximately 1.2 or more times, approximately 1.25 or more times, approximately 1.3 or more times, approximately 1.35 or more times, approximately 1.4 or more times, approximately 1.45 or more times, approximately 1.5 or more times, approximately 1.6 or more times, approximately 1.8 or more times, approximately 2 or more times, approximately 2.2 or more times, approximately 2.4 or more times, approximately 2.6 or more times, approximately 2.8 or more times, approximately 3 or more times, approximately 3.2 or more times, approximately 3.4 or more times, approximately 3.6 or more times, approximately 3.8 or more times, approximately 4 or more times, or approximately 4.2 or more times compared to the content of RebF in a control extract.

In some embodiments, the content of RebG in the extract of the present invention in relation to the chemical feature (h') is approximately 1.2 or more times, approximately 1.3 or more times, approximately 1.4 or more times, approximately 1.5 or more times, approximately 2 or more times, approximately 2.5 or more times, approximately 3 or more times, approximately 3.5 or more times, approximately 4 or more times, approximately 4.5 or more times, approximately 5 or more times, approximately 6 or more times, approximately 7 or more times, approximately 8 or more times, approximately 9 or more times, approximately 10 or more times, approximately 12.5 or more times, approximately 15 or more times, approximately 17.5 or more times, approximately 20 or more times, approximately 22.5 or more times, approximately 25 or more times, approximately 30 or more times, approximately 35 or more times, approximately 40 or more times, approximately 45 or more times, or approximately 50 or more times compared to the content of RebG in a control extract.

In some embodiments, the content of RebM in the extract of the present invention in relation to the chemical feature (i') is approximately 1.05 or more times, approximately 1.1 or more times, approximately 1.15 or more times, approximately 1.2 or more times, approximately 1.3 or more times, approximately 1.4 or more times, approximately 1.5 or more times, approximately 1.7 or more times, approximately 2 or more times, approximately 2.5 or more times, approximately 3 or more times, approximately 3.5 or more times, approximately 4 or more times, approximately 4.5 or more times, approximately 5 or more times, approximately 6 or more times, approximately 7 or more times, approximately 8 or more times, approximately 9 or more times, approximately 10 or more times, approximately 12 or more times, approximately 14 or more times, approximately 16 or more times, approximately 18 or more times, approximately 20 or more times, approximately 22 or more times, or approximately 24 or more times compared to the content of RebM in a control extract.

In some embodiments, the content of a steviol glycoside having a β-1,3-glucoside bond in the extract of the present invention in relation to the chemical feature (j') is approximately 1.02 or more times, approximately 1.03 or more times, approximately 1.04 or more times, approximately 1.05 or more times, approximately 1.1 or more times, approximately 1.15 or more times, approximately 1.2 or more times, approximately 1.3 or more times, approximately 1.4 or more times, approximately 1.5 or more times, approximately 2 or more times, approximately 2.5 or more times, approximately 3 or more times, approximately 3.5 or more times, approximately 4 or more times, approximately 4.5 or more times, approximately 5 or more times, approximately 6 or more times, approximately 7 or more times, approximately 8 or more times, approximately 9 or more times, approximately 10 or more times, approximately 12.5 or more times, approximately 15 or more times, approximately 17.5 or more times, approximately 20 or more times, approximately 22.5 or more times, approximately 25 or more times, approximately 30 or more times, approximately 35 or more times, approximately 40 or more times, approximately 45 or more times, or approximately 50 or more times compared to the content of the steviol glycoside having a β-1,3-glucoside bond in a control extract.

**In** some embodiments, the content of RebD in the extract of the present invention in relation to the chemical feature (k') is approximately 0.8 or less times, approximately 0.76 or less times, approximately 0.72 or less times, approximately 0.68 or less times, approximately 0.64 or less times, approximately 0.6 or less times, approximately 0.56 or less times, approximately 0.52 or less times, approximately 0.5 or less times, approximately 0.48 or less times, approximately 0.46 or less times, approximately 0.44 or less times, approximately 0.42 or less times, approximately 0.4 or less times, approximately 0.38 or less times, approximately 0.36 or less times, approximately 0.34 or less times, approximately 0.32 or less times, approximately 0.3 or less times, approximately 0.28 or less times, approximately 0.26 or less times, approximately 0.24 or less times, approximately 0.22 or less times, approximately 0.2 or less times, approximately 0.18 or less times, approximately 0.16 or less times, or approximately 0.14 or less times compared to the content of RebD in a control extract.

In some embodiments, the content of RebE in the extract of the present invention in relation to the chemical feature (l') is approximately 0.6 or less times, approximately 0.56 or less times, approximately 0.52 or less times, approximately 0.48 or less times, approximately 0.44 or less times, approximately 0.4 or less times, approximately 0.38 or less times, approximately 0.36 or less times, approximately 0.34 or less times, approximately 0.32 or less times, approximately 0.3 or less times, approximately 0.28 or less times, approximately 0.26 or less times, approximately 0.24 or less times, approximately 0.22 or less times, approximately 0.2 or less times, approximately 0.18 or less times, approximately 0.16 or less times, approximately 0.14 or less times, approximately 0.12 or less times, approximately 0.1 or less times, approximately 0.08 or less times, approximately 0.06 or less times, approximately 0.04 or less times, approximately 0.03 or less times, approximately 0.02 or less times, or approximately 0.01 or less times compared to the content of RebE in a control extract.

In some embodiments, the content of RebN in the extract of the present invention in relation to the chemical feature (m') is approximately 0.9 or less times, approximately 0.86 or less times, approximately 0.82 or less times, approximately 0.78 or less times, approximately 0.74 or less times, approximately 0.7 or less times, approximately 0.66 or less times, approximately 0.62 or less times, approximately 0.58 or less times, approximately 0.54 or less times, approximately 0.5 or less times, approximately 0.48 or less times, approximately 0.46 or less times, approximately 0.44 or less times, approximately 0.42 or less times, approximately 0.4 or less times, approximately 0.38 or less times, approximately 0.36 or less times, approximately 0.34 or less times, approximately 0.32 or less times, approximately 0.3 or less times, approximately 0.28 or less times, approximately 0.26 or less times, approximately 0.24 or less times, approximately 0.22 or less times, approximately 0.2 or less times, or approximately 0.18 or less times compared to the content of RebN in a control extract.

In some embodiments, the content of stevioside in the extract of the present invention in relation to the chemical feature (n') is approximately 0.8 or less times, approximately 0.75 or less times, approximately 0.7 or less times, approximately 0.65 or less times, approximately 0.6 or less times, approximately 0.55 or less times, approximately 0.5 or less times, approximately 0.45 or less times, approximately 0.4 or less times, approximately 0.35 or less times, approximately 0.3 or less times, approximately 0.28 or less times, approximately 0.26 or less times, approximately 0.24 or less times, approximately 0.22 or less times, approximately 0.2 or less times, approximately 0.18 or less times, approximately 0.16 or less times, approximately 0.14 or less times, approximately 0.12 or less times, approximately 0.1 or less times, approximately 0.08 or less times, approximately 0.06 or less times, approximately 0.05 or less times, approximately 0.04 or less times, approximately 0.03 or less times, or approximately 0.02 or less times compared to the content of stevioside in a control extract.

In some embodiments, the content of a steviol glycoside having no β-1,3-glucoside bond in the extract of the present invention in relation to the chemical feature (o') is approximately 0.9 or less times, approximately 0.86 or less times, approximately 0.82 or less times, approximately 0.78 or less times, approximately 0.74 or less times, approximately 0.7 or less times, approximately 0.66 or less times, approximately 0.62 or less times, approximately 0.6 or less times, approximately 0.56 or less times, approximately 0.52 or less times, approximately 0.48 or less times, approximately 0.44 or less times, approximately 0.4 or less times, approximately 0.38 or less times, approximately 0.36 or less times, approximately 0.34 or less times, approximately 0.32 or less times, approximately 0.3 or less times, approximately 0.28 or less times, approximately 0.26 or less times, approximately 0.24 or less times, approximately 0.22 or less times, approximately 0.2 or less times, approximately 0.18 or less times, approximately 0.16 or less times, approximately 0.14 or less times, approximately 0.12 or less times, approximately 0.1 or less times, approximately 0.08 or less times, approximately 0.06 or less times, approximately 0.04 or less times, approximately 0.03 or less times, approximately 0.02 or less times, or approximately 0.01 or less times compared to the content of the steviol glycoside having no β-1,3-glucoside bond in a control extract.

In some embodiments, the content ratio of any one of RebA, RebB, RebF, RebG, and RebM to any one of RebD, RebE, RebN, and stevioside in the extract of the present invention in relation to the chemical feature (p') is approximately 1.2 or more times, approximately 1.5 or more times, approximately 2 or more times, approximately 2.5 or more times, approximately 3 or more times, approximately 3.5 or more times, approximately 4 or more times, approximately 4.5 or more times, approximately 5 or more times, approximately 10 or more times, approximately 15 or more times, approximately 20 or more times, approximately 30 or more times, approximately 40 or more times, approximately 50 or more times, approximately 60 or more times, approximately 70 or more times, approximately 80 or more times, approximately 90 or more times, approximately 100 or more times, approximately 150 or more times, approximately 200 or more times, approximately 250 or more times, approximately 300 or more times, approximately 400 or more times, approximately 500 or more times, approximately 600 or more times, or approximately 700 or more times compared to the above ratio in a control extract.

In some embodiments, the content ratio of a steviol glycoside having a β-1,3-glucoside bond to a steviol glycoside having no β-1,3-glucoside bond in the extract of the present invention in relation to the chemical feature (q') is approximately 1.2 or more times, approximately 1.5 or more times, approximately 2 or more times, approximately 2.5 or more times, approximately 3 or more times, approximately 3.5 or more times, approximately 4 or more times, approximately 4.5 or more times, approximately 5 or more times, approximately 10 or more times, approximately 15 or more times, approximately 20 or more times, approximately 30 or more times, approximately 40 or more times, approximately 50 or more times, approximately 60 or more times, approximately 70 or more times, approximately 80 or more times, approximately 90 or more times, approximately 100 or more times, approximately 150 or more times, approximately 200 or more times, approximately 250 or more times, approximately 300 or more times, approximately 400 or more times, approximately 500 or more times, approximately 600 or more times, or approximately 700 or more times compared to the above ratio in a control extract.

In some embodiments, the content ratio of a steviol glycoside produced by UGT76G1 from its substrate steviol glycoside to the substrate steviol glycoside of UGT76G1 in the extract of the present invention in relation to the chemical feature (r') is approximately 1.2 or more times, approximately 1.5 or more times, approximately 2 or more times, approximately 3 or more times, approximately 4 or more times, approximately 5 or more times, approximately 6 or more times, approximately 8 or more times, approximately 10 or more times, approximately 15 or more times, approximately 20 or more times, approximately 25 or more times, approximately 30 or more times, approximately 35 or more times, approximately 40 or more times, approximately 45 or more times, approximately 50 or more times, approximately 55 or more times, approximately 60 or more times, approximately 65 or more times, approximately 70 or more times, approximately 75 or more times, approximately 80 or more times, approximately 85 or more times, approximately 90 or more times, approximately 95 or more times, or approximately 100 or more times compared to the above ratio in a control extract.

In some embodiments, the content ratio of RebM to RebD in the extract of the present invention in relation to the chemical feature (a') is approximately 0.12 or more, approximately 0.16 or more, approximately 0.2 or more, approximately 0.24 or more, approximately 0.28 or more, approximately 0.32 or more, approximately 0.36 or more, approximately 0.4 or more, approximately 0.44 or more, approximately 0.48 or more, approximately 0.5 or more, approximately 0.525 or more, approximately 0.55 or more, approximately 0.575 or more, approximately 0.6 or more, approximately 0.625 or more, approximately 0.65 or more, approximately 0.675 or more, approximately 0.7 or more, approximately 0.725 or more, approximately 0.75 or more, approximately 0.775 or more, approximately 0.8 or more, approximately 0.825 or more, approximately 0.85 or more, approximately 0.875 or more, approximately 0.9 or more, approximately 0.925 or more, approximately 0.95 or more, approximately 0.975 or more, or approximately 1 or more.

In some embodiments, the content ratio of RebB to RebE in the extract of the present invention in relation to the chemical feature (b') is approximately 0.17 or more, approximately 0.5 or more, approximately 1 or more, approximately 1.5 or more, approximately 2 or more, approximately 2.5 or more, approximately 3 or more, approximately 3.5 or more, approximately 4 or more, approximately 4.5 or more, approximately 5 or more, approximately 5.5 or more, approximately 6 or more, approximately 6.5 or more, approximately 7 or more, approximately 7.5 or more, approximately 8 or more, approximately 8.5 or more, approximately 9 or more, approximately 9.5 or more, approximately 10 or more, approximately 10.5 or more, approximately 11 or more, approximately 11.5 or more, approximately 12 or more, approximately 12.5 or more, approximately 13 or more, approximately 13.5 or more, approximately 14 or more, approximately 14.5 or more, or approximately 15 or more.

In some embodiments, the content ratio of RebG to RebE in the extract of the present invention in relation to the chemical feature (c') is approximately 0.004 or more, approximately 0.05 or more, approximately 0.1 or more, approximately 0.2 or more, approximately 0.3 or more, approximately 0.4 or more, approximately 0.5 or more, approximately 0.6 or more, approximately 0.7 or more, approximately 0.8 or more, approximately 0.9 or more, approximately 1 or more, approximately 1.05 or more, approximately 1.07 or more, approximately 1.1 or more, approximately 1.12 or more, approximately 1.13 or more, approximately 1.17 or more, approximately 1.2 or more, approximately 1.25 or more, approximately 1.3 or more, approximately 1.35 or more, approximately 1.37 or more, approximately 1.38 or more, approximately 1.4 or more, approximately 1.5 or more, approximately 1.6 or more, approximately 1.7 or more, approximately 1.72 or more, approximately 1.75 or more, approximately 1.8 or more, approximately 1.85 or more, approximately 1.9 or more, approximately 1.95 or more, or approximately 2 or more.

In some embodiments, the content ratio of RebG to stevioside in the extract of the present invention in relation to the chemical feature (d') is approximately 0.0007 or more, approximately 0.004 or more, approximately 0.006 or more, approximately 0.008 or more, approximately 0.01 or more, approximately 0.012 or more, approximately 0.014 or more, approximately 0.016 or more, approximately 0.018 or more, approximately 0.02 or more, approximately 0.025 or more, approximately 0.03 or more, approximately 0.035 or more, approximately 0.04 or more, approximately 0.045 or more, approximately 0.05 or more, approximately 0.055 or more, approximately 0.06 or more, approximately 0.065 or more, approximately 0.07 or more, approximately 0.075 or more, approximately 0.08 or more, approximately 0.085 or more, approximately 0.09 or more, approximately 0.091 or more, approximately 0.092 or more, approximately 0.093 or more, approximately 0.094 or more, approximately 0.095 or more, approximately 0.096 or more, approximately 0.097 or more, approximately 0.098 or more, approximately 0.099 or more, or approximately 0.1 or more.

In some embodiments, the content of RebA in the extract of the present invention in relation to the chemical feature (e') is approximately 2% by weight or more, approximately 2.15% by weight or more, approximately 2.3% by weight or more, approximately 2.45% by weight or more, approximately 2.6% by weight or more, approximately 2.75% by weight or more, approximately 2.9% by weight or more, approximately 3.05% by weight or more, approximately 3.2% by weight or more, approximately 3.35% by weight or more, approximately 3.5% by weight or more, approximately 3.65% by weight or more, approximately 3.8% by weight or more, approximately 4% by weight or more, approximately 4.2% by weight or more, approximately 4.4% by weight or more, approximately 4.6% by weight or more, approximately 4.8% by weight or more, approximately 5% by weight or more, approximately 5.25% by weight or more, approximately 5.5% by weight or more, approximately 5.75% by weight or more, approximately 6% by weight or more, approximately 6.25% by weight or more, approximately 6.5% by weight or more, approximately 6.75% by weight or more or approximately 7% by weight or more.

In some embodiments, the content of RebB in the extract of the present invention in relation to the chemical feature (f) is approximately 0.04% by weight or more, approximately 0.044% by weight or more, approximately 0.048% by weight or more, approximately 0.052% by weight or more, approximately 0.056% by weight or more, approximately 0.06% by weight or more, approximately 0.064% by weight or more, approximately 0.068% by weight or more, approximately 0.072% by weight or more, approximately 0.076% by weight or more, approximately 0.08% by weight or more, approximately 0.084% by weight or more, approximately 0.088% by weight or more, approximately 0.092% by weight or more, approximately 0.096% by weight or more, approximately 0.1% by weight or more, approximately 0.105% by weight or more, approximately 0.11% by weight or more, approximately 0.115% by weight or more, approximately 0.12% by weight or more, approximately 0.125% by weight or more, approximately 0.13% by weight or more, approximately 0.135% by weight or more, approximately 0.14% by weight or more, approximately 0.145% by weight or more, approximately 0.15% by weight or more, or approximately 0.16% by weight or more.

In some embodiments, the content of RebF in the extract of the present invention in relation to the chemical feature (g') is approximately 0.06% by weight or more, approximately 0.064% by weight or more, approximately 0.068% by weight or more, approximately 0.072% by weight or more, approximately 0.076% by weight or more, approximately 0.08% by weight or more, approximately 0.084% by weight or more, approximately 0.088% by weight or more, approximately 0.092% by weight or more, approximately 0.096% by weight or more, approximately 0.1% by weight or more, approximately 0.105% by weight or more, approximately 0.11% by weight or more, approximately 0.115% by weight or more, approximately 0.12% by weight or more, approximately 0.125% by weight or more, approximately 0.13% by weight or more, approximately 0.135% by weight or more, approximately 0.14% by weight or more, approximately 0.145% by weight or more, approximately 0.15% by weight or more, approximately 0.155% by weight or more, approximately 0.16% by weight or more, approximately 0.17% by weight or more, approximately 0.18% by weight or more, approximately 0.19% by weight or more, or approximately 0.2% by weight or more.

In some embodiments, the content of RebG in the extract of the present invention in relation to the chemical feature (h') is approximately 0.001% by weight or more, approximately 0.002% by weight or more, approximately 0.003% by weight or more, approximately 0.004% by weight or more, approximately 0.005% by weight or more, approximately 0.006% by weight or more, approximately 0.007% by weight or more, approximately 0.008% by weight or more, approximately 0.009% by weight or more, approximately 0.01% by weight or more, approximately 0.011% by weight or more, approximately 0.012% by weight or more, approximately 0.013% by weight or more, approximately 0.014% by weight or more, approximately 0.016% by weight or more, approximately 0.018% by weight or more, approximately 0.02% by weight or more, approximately 0.022% by weight or more, approximately 0.024% by weight or more, approximately 0.026% by weight or more, approximately 0.028% by weight or more, approximately 0.03% by weight or more, approximately 0.032% by weight or more, approximately 0.034% by weight or more, approximately 0.036% by weight or more, approximately 0.038% by weight or more, or approximately 0.04% by weight or more.

In some embodiments, the content of RebM in the extract of the present invention in relation to the chemical feature (i') is approximately 0.15% by weight or more, approximately 0.17% by weight or more, approximately 0.19% by weight or more, approximately 0.21% by weight or more, approximately 0.23% by weight or more, approximately 0.25% by weight or more, approximately 0.27% by weight or more, approximately 0.29% by weight or more, approximately 0.31% by weight or more, approximately 0.33% by weight or more, approximately 0.35% by weight or more, approximately 0.37% by weight or more, approximately 0.39% by weight or more, approximately 0.41% by weight or more, approximately 0.43% by weight or more, approximately 0.45% by weight or more, approximately 0.47% by weight or more, approximately 0.49% by weight or more, approximately 0.51% by weight or more, approximately 0.54% by weight or more, approximately 0.57% by weight or more, approximately 0.6% by weight or more, approximately 0.63% by weight or more, approximately 0.66% by weight or more, approximately 0.69% by weight or more, approximately 0.72% by weight or more, or approximately 0.75% by weight or more.

In some embodiments, the content of a steviol glycoside having a β-1,3-glucoside bond in the extract of the present invention in relation to the chemical feature (j') is approximately 0.001% by weight or more, approximately 0.002% by weight or more, approximately 0.005% by weight or more, approximately 0.01% by weight or more, approximately 0.02% by weight or more, approximately 0.04% by weight or more, approximately 0.06% by weight or more, approximately 0.08% by weight or more, approximately 0.1% by weight or more, approximately 0.15% by weight or more, approximately 0.2% by weight or more, approximately 0.3% by weight or more, approximately 0.4% by weight or more, approximately 0.5% by weight or more, approximately 0.6% by weight or more, approximately 0.7% by weight or more, approximately 0.8% by weight or more, approximately 1% by weight or more, approximately 1.5% by weight or more, approximately 2% by weight or more, approximately 2.5% by weight or more, approximately 3% by weight or more, approximately 3.5% by weight or more, approximately 4% by weight or more, approximately 5% by weight or more, approximately 6% by weight or more, or approximately 7% by weight or more.

In some embodiments, the content of RebD in the extract of the present invention in relation to the chemical feature (k') is approximately 1.65% by weight or less, approximately 1.6% by weight or less, approximately 1.55% by weight or less, approximately 1.5% by weight or less, approximately 1.45% by weight or less, approximately 1.4% by weight or less, approximately 1.35% by weight or less, approximately 1.3% by weight or less, approximately 1.25% by weight or less, approximately 1.2% by weight or less, approximately 1.15% by weight or less, approximately 1.1% by weight or less, approximately 1.05% by weight or less, approximately 1% by weight or less, approximately 0.95% by weight or less, approximately 0.9% by weight or less, approximately 0.85% by weight or less, approximately 0.8% by weight or less, approximately 0.75% by weight or less, approximately 0.7% by weight or less, approximately 0.65% by weight or less, approximately 0.6% by weight or less, approximately 0.55% by weight or less, approximately 0.5% by weight or less, approximately 0.45% by weight or less, approximately 0.4% by weight or less, or approximately 0.35% by weight or less.

In some embodiments, the content of RebE in the extract of the present invention in relation to the chemical feature (l') is approximately 0.34% by weight or less, approximately 0.32% by weight or less, approximately 0.3% by weight or less, approximately 0.28% by weight or less, approximately 0.26% by weight or less, approximately 0.24% by weight or less, approximately 0.22% by weight or less, approximately 0.2% by weight or less, approximately 0.18% by weight or less, approximately 0.16% by weight or less, approximately 0.14% by weight or less, approximately 0.12% by weight or less, approximately 0.1% by weight or less, approximately 0.08% by weight or less, approximately 0.06% by weight or less, approximately 0.05% by weight or less, approximately 0.04% by weight or less, approximately 0.03% by weight or less, approximately 0.02% by weight or less, approximately 0.015% by weight or less, approximately 0.01% by weight or less, approximately 0.009% by weight or less, approximately 0.008% by weight or less, approximately 0.007% by weight or less, approximately 0.006% by weight or less, approximately 0.005% by weight or less, or approximately 0.004% by weight or less.

In some embodiments, the content of RebN in the extract of the present invention in relation to the chemical feature (m') is approximately 0.35% by weight or less, approximately 0.33% by weight or less, approximately 0.31% by weight or less, approximately 0.29% by weight or less, approximately 0.27% by weight or less, approximately 0.25% by weight or less, approximately 0.24% by weight or less, approximately 0.23% by weight or less, approximately 0.22% by weight or less, approximately 0.21% by weight or less, approximately 0.2% by weight or less, approximately 0.19% by weight or less, approximately 0.18% by weight or less, approximately 0.17% by weight or less, approximately 0.16% by weight or less, approximately 0.15% by weight or less, approximately 0.14% by weight or less, approximately 0.13% by weight or less, approximately 0.12% by weight or less, approximately 0.11% by weight or less, approximately 0.1% by weight or less, approximately 0.09% by weight or less, approximately 0.08% by weight or less, approximately 0.07% by weight or less, approximately 0.06% by weight or less, approximately 0.055% by weight or less, or approximately 0.05% by weight or less.

In some embodiments, the content of stevioside in the extract of the present invention in relation to the chemical feature (n') is approximately 2.5% by weight or less, approximately 2.4% by weight or less, approximately 2.3% by weight or less, approximately 2.2% by weight or less, approximately 2.1% by weight or less, approximately 2% by weight or less, approximately 1.9% by weight or less, approximately 1.8% by weight or less, approximately 1.7% by weight or less, approximately 1.6% by weight or less, approximately 1.5% by weight or less, approximately 1.4% by weight or less, approximately 1.3% by weight or less, approximately 1.2% by weight or less, approximately 1.1% by weight or less, approximately 1% by weight or less, approximately 0.9% by weight or less, approximately 0.8% by weight or less, approximately 0.7% by weight or less, approximately 0.6% by weight or less, approximately 0.5% by weight or less, approximately 0.45% by weight or less, approximately 0.4% by weight or less, approximately 0.35% by weight or less, approximately 0.3% by weight or less, approximately 0.25% by weight or less, or approximately 0.2% by weight or less.

In some embodiments, the content of steviol glycoside having no β-1,3-glucoside bond in the extract of the present invention in relation to the chemical feature (o') is approximately 2.5% by weight or less, approximately 2.2% by weight or less, approximately 2% by weight or less, approximately 1.8% by weight or less, approximately 1.6% by weight or less, approximately 1.4% by weight or less, approximately 1.2% by weight or less, approximately 1% by weight or less, approximately 0.9% by weight or less, approximately 0.8% by weight or less, approximately 0.7% by weight or less, approximately 0.6% by weight or less, approximately 0.5% by weight or less, approximately 0.4% by weight or less, approximately 0.3% by weight or less, approximately 0.2% by weight or less, approximately 0.1% by weight or less, approximately 0.08% by weight or less, approximately 0.06% by weight or less, approximately 0.04% by weight or less, approximately 0.02% by weight or less, approximately 0.015% by weight or less, approximately 0.01% by weight or less, approximately 0.008% by weight or less, approximately 0.006% by weight or less, approximately 0.005% by weight or less, or approximately 0.004% by weight or less.

In some embodiments, the content ratio of any one of RebA, RebB, RebF, RebG, and RebM to any one of RebD, RebE, RebN, and stevioside in the extract of the present invention in relation to the chemical feature (p') is approximately 0.0007 or more, approximately 0.004 or more, approximately 0.01 or more, approximately 0.02 or more, approximately 0.03 or more, approximately 0.05 or more, approximately 0.08 or more, approximately 0.091 or more, approximately 0.095 or more, approximately 0.1 or more, approximately 0.2 or more, approximately 0.3 or more, approximately 0.5 or more, approximately 0.8 or more, approximately 1 or more, approximately 1.05 or more, approximately 1.07 or more, approximately 1.1 or more, approximately 1.2 or more, approximately 1.5 or more, approximately 2 or more, approximately 3 or more, approximately 4 or more, approximately 5 or more, approximately 8 or more, approximately 10 or more, approximately 10.5 or more, approximately 11 or more, approximately 11.5 or more, approximately 12 or more, approximately 12.5 or more, approximately 13 or more, approximately 13.5 or more, approximately 14 or more, approximately 14.5 or more, or approximately 15 or more.

In some embodiments, the content ratio of a steviol glycoside having a β-1,3-glucoside bond to a steviol glycoside having no β-1,3-glucoside bond in the extract of the present invention in relation to the chemical feature (q') is approximately 0.0007 or more, approximately 0.004 or more, approximately 0.01 or more, approximately 0.02 or more, approximately 0.03 or more, approximately 0.05 or more, approximately 0.08 or more, approximately 0.091 or more, approximately 0.095 or more, approximately 0.1 or more, approximately 0.2 or more, approximately 0.3 or more, approximately 0.5 or more, approximately 0.8 or more, approximately 1 or more, approximately 1.05 or more, approximately 1.07 or more, approximately 1.1 or more, approximately 1.2 or more, approximately 1.5 or more, approximately 2 or more, approximately 3 or more, approximately 4 or more, approximately 5 or more, approximately 8 or more, approximately 10 or more, approximately 10.5 or more, approximately 11 or more, approximately 11.5 or more, approximately 12 or more, approximately 12.5 or more, approximately 13 or more, approximately 13.5 or more, approximately 14 or more, approximately 14.5 or more, or approximately 15 or more.

In some embodiments, the content ratio of a steviol glycoside produced by UGT76G1 from its substrate steviol glycoside to the substrate steviol glycoside of UGT76G1 in the extract of the present invention in relation to the chemical feature (r') is approximately 0.15 or more, approximately 0.3 or more, approximately 0.5 or more, approximately 0.7 or more, approximately 0.9 or more, approximately 1 or more, approximately 1.5 or more, approximately 2 or more, approximately 3 or more, approximately 5 or more, approximately 7 or more, approximately 10 or more, approximately 15 or more, approximately 16 or more, approximately 17 or more, approximately 20 or more, approximately 25 or more, approximately 30 or more, approximately 35 or more, approximately 40 or more, approximately 45 or more, approximately 50 or more, approximately 51 or more, approximately 55 or more, approximately 60 or more, approximately 65 or more, approximately 66 or more, approximately 70 or more, approximately 75 or more, or approximately 80 or more.

In a preferable embodiment, the contents or content ratios of the steviol glycosides related to the chemical features (a') to (r') described above are those in the unpurified extract (crude extract) of the present invention.

The extract of the present invention thus obtained and/or a steviol glycoside obtained by the steviol glycoside production method of the present invention can be mixed with other component(s) to produce a food, sweetener composition, flavor or medicament comprising the steviol glycoside (hereinafter, may be generically referred to as the food, etc.). Accordingly, the present invention further provides a method of producing a food, etc., comprising a step of mixing the extract of the present invention, and/or a steviol glycoside obtained by the steviol glycoside production method of the present invention with other component(s). The present invention further provides a food, etc. obtained by the production method. In this context, the food encompasses a beverage. In some embodiments, the food, etc. do not comprise a steviol glycoside other than the steviol glycoside contained in the extract of the present invention and/or the steviol glycoside obtained by the steviol glycoside production method of the present invention.

The food, etc., when comprising the extract of the present invention, can have similar chemical feature(s) to that of the extract of the present invention. In some embodiments, the food, etc. has the same steviol glycoside content ratio as that in at least one of the chemical features (a') to (d'), (p') and (r'). For example, a food, etc. having the same steviol glycoside content ratio as that in the chemical feature (a') has a higher (e.g., approximately 1.2 or more times, approximately 1.6 or more times, approximately 2 or more times, approximately 2.4 or more times, approximately 2.8 or more times, approximately 3.2 or more times, approximately 3.6 or more times, approximately 4 or more times, approximately 4.4 or more times, approximately 4.8 or more times, approximately 5.2 or more times, approximately 5.6 or more times, approximately 6 or more times, approximately 6.4 or more times, approximately 6.8 or more times, approximately 7.2 or more times, approximately 7.6 or more times, approximately 8 or more times, approximately 8.4 or more times, approximately 8.8 or more times, approximately 9.2 or more times, approximately 9.6 or more times, approximately 10 or more times, approximately 10.5 or more times, approximately 11 or more times, approximately 11.5 or more times or approximately 12 or more times higher) content ratio of RebM to RebD as compared with a food, etc. produced in the same manner using the same components except that an extract of a stevia plant that does not have the genetic feature(s) of the present invention is included instead of the extract of the present invention, and can have a content ratio of RebM to RebD of approximately 0.12 or more, approximately 0.16 or more, approximately 0.2 or more, approximately 0.24 or more, approximately 0.28 or more, approximately 0.32 or more, approximately 0.36 or more, approximately 0.4 or more, approximately 0.44 or more, approximately 0.48 or more, approximately 0.5 or more, approximately 0.525 or more, approximately 0.55 or more, approximately 0.575 or more, approximately 0.6 or more, approximately 0.625 or more, approximately 0.65 or more, approximately 0.675 or more, approximately 0.7 or more, approximately 0.725 or more, approximately 0.75 or more, approximately 0.775 or more, approximately 0.8 or more, approximately 0.825 or more, approximately 0.85 or more, approximately 0.875 or more, approximately 0.9 or more, approximately 0.925 or more, approximately 0.95 or more, approximately 0.975 or more, or approximately 1 or more.

### 5. Nucleotide sequence relating to plant of present invention

In another aspect, the present invention provides nucleotide sequences relating to the stevia plant of the present invention. A specific embodiment of nucleotide sequences relating to a stevia plant having the genetic feature (1) of the present invention comprises or consists of a nucleotide sequence selected from SEQ ID NOs: 10 to 13 and 50. A specific embodiment of nucleotide sequences relating to a stevia plant having the genetic feature (2) of the present invention comprises or consists of a nucleotide sequence selected from SEQ ID NOs: 14 to 17 and 52. A specific embodiment of nucleotide sequences relating to a stevia plant having the genetic feature (3) of the present invention comprises or consists of a nucleotide sequence selected from SEQ ID NOs: 18 to 21 and 54.

### 6. Dead tissue or dead cell obtained of present invention

In a further aspect, the present invention provides a dead tissue or cell of the stevia plant of the present invention, a stevia plant selected by the screening method of the present invention or a stevia plant produced by the production method of the present invention (hereinafter, may be referred to as the "dead tissue or dead cell of the present invention"). The term "dead" means a state having no ability to propagate, proliferate, regenerate or grow, and includes both the case of dying naturally without an artificial operation and the case of dying by an artificial operation such as cutting, homogenization, heating (including heating with air or other gasses, heating with water or other liquid, and heating with steam or other vapors), freezing, drying, and freeze drying. The dead tissue means a tissue in which all cells contained are dead and includes a dead plant. Specific examples of the dead tissue or dead cell of the present invention include dead tissues or cells except for seeds. More specifically, examples thereof include dead embryos, meristem cells, pollens, leaves, roots, root apices, petals, protoplasts, leaf sections and calluses. A dried leaf is preferred.

The dead tissue or dead cells can be used as raw materials for the above extracts, steviol glycoside purified products, pharmaceuticals, flavorings, food and beverages, etc.

In this aspect, the present invention provides a method of producing an extract of a dead tissue or a dead cell, comprising obtaining an extract from the dead tissue or dead cell of the present invention by the same method as the extract production method of the present invention. The present invention further provides a method of producing a steviol glycoside having a β-1,3-glucoside bond, comprising a step of purifying the steviol glycoside having a β-1,3-glucoside bond from the obtained extract.

The present invention also provides an extract of the dead tissue or dead cell of the present invention, and a method of producing a food, a sweetener composition, a flavor or a medicament, comprising: a step of providing an extract obtained by the method for producing a dead tissue or dead cell extract; and a step of adding the extract to a raw material for the food, sweetener composition, flavor or medicament.

### [Examples]

Hereinafter, Examples regarding the present invention will be described. However, the present invention is not limited by these specific embodiments.

### (1) Generation of segregating population

Commercially available stevia plants were selected and crossed over three generations by focusing on the content of RebD or RebM to obtain population A with high RebD content and population B with high RebM content. Further, the populations A and B were crossed to obtain segregating population C.

### (2) Gene analysis of individuals with high RebM content

An appropriate amount of fresh leaves was sampled from each grown individual in the population C, and the concentration of steviol glycoside was quantitatively determined by LC-MS/MS (Shimadzu LCMS8050). Specifically, 0.25 g of the fresh leaves was dried by freeze drying, and 0.05 g of homogenized dry matter thereof was added into a 100-fold amount (5 mL) of pure water. Extraction by ultrasonic treatment for 20 minutes, and centrifugation and filtration were performed, followed by 60-fold dilution with 32% acetonitrile to obtain a liquid sample. The concentration of RebA, RebB, RebC, RebD, RebE, RebF, RebG, RebM, RebN and stevioside was quantitatively determined by LC/MS-MS analysis on this 1 mL of liquid sample in an LCMS8050 MRM mode. Further, genomic DNA was extracted from the fresh leaves of some individuals in the population C, and genetically analyzed using a sequencer (HiSeq 2500, Illumina, Inc.). The results are shown in Tables 2 to 3. In the tables, the numeric values of steviol glycosides represent % by weight in dried leaves, and the genetic features are indicated by "+" for positivity and "-" for negativity. "STV" denotes stevioside, and "TSG" denotes total steviol glycoside. The numeric values of TSG are total values of all the steviol glycosides measured (i.e., RebA, RebB, RebC, RebD, RebE, RebF, RebG, RebM, RebN and stevioside).

**[Table 2-1]**

| Table 2: Genetic feature and steviol glycoside content in each individual in population C - (1) | | | | | | | |
|---|---|---|---|---|---|---|---|
| | RebA | RebB | RebC | RebD | STV | RebF | RebM |
| C1 | 3. 786502 | 0.077078 | 0.24346 | 1.522212 | 2.209915 | 0,139107 | 0.219286 |
| C2 | 3. 924214 | 0.081276 | 0.194904 | 0. 774268 | 0. 715449 | 0.120277 | 0.304381 |
| C3 | 4. 770307 | 0.1019 | 0. 224913 | 0. 96954 | 0.824279 | 0.14032 | 0.38172 |
| C4 | 3. 356523 | 0. 060404 | 0. 200702 | 1. 069825 | 1.638942 | 0.116546 | 0.167597 |
| C5 | 3. 513728 | 0.083856 | 0. 240683 | 1. 162947 | 1. 974361 | 0.126278 | 0.195132 |
| C6 | 4.251365 | 0.088944 | 0.192123 | 0.187532 | 0. 465366 | 0. 128627 | 0.4412 |
| C7 | 4. 583756 | 0.069368 | 0.192163 | 0.536089 | 0.403402 | 0. 120166 | 0.289563 |
| C8 | 4. 721994 | 0. 097663 | 0.205402 | 0.743849 | 0. 479511 | 0.126674 | 0.401899 |
| C9 | 3.095679 | 0.064023 | 0.180796 | 1. 113914 | 1. 266811 | 0.100967 | 0.168674 |
| C10 | 2. 140972 | 0.035659 | 0.090451 | 0.686378 | 0. 273428 | 0.061744 | 0. 339339 |
| C11 | 3. 15986 | 0.041142 | 0. 158603 | 0.584393 | 0.300795 | 0. 090574 | 0.290687 |
| C12 | 3. 190831 | 0.071139 | 0. 233477 | 1. 670157 | 1. 98177 | 0. 125778 | 0.242854 |
| C13 | 3. 393655 | 0. 049451 | 0.155243 | 0.420731 | 0.374815 | 0.1022 | 0. 207204 |
| C14 | 2. 860136 | 0.051874 | 0.191115 | 1.578658 | 1. 410705 | 0.123791 | 0. 25605 |
| C15 | 5. 691578 | 0.12385 | 0. 298395 | 1. 248008 | 0.887889 | 0.168764 | 0.537598 |
| C16 | 3. 125041 | 0.053355 | 0.209145 | 1.275028 | 1.647713 | 0.105982 | 0.225176 |
| C17 | 3. 347205 | 0. 063993 | 0.16953 | 0. 485618 | 0.52877 | 0.084103 | 0. 242361 |
| C18 | 3.386862 | 0.068342 | 0.219082 | 1.037035 | 1. 367305 | 0.097393 | 0.228556 |
| C19 | 3.648149 | 0.05497 | 0. 173458 | 0.574277 | 0.341452 | 0. 09063 | 0.414205 |
| C20 | 5. 606061 | 0.09929 | 0. 250488 | 0. 789457 | 0.612033 | 0. 13986 | 0.46294 |
| C21 | 3. 853469 | 0.084685 | 0.234509 | 1. 165485 | 1. 430984 | 0. 115418 | 0.270798 |
| C22 | 4. 849616 | 0.081854 | 0.225445 | 0. 694839 | 0.437815 | 0.142207 | 0.453284 |
| C23 | 3. 269713 | 0.066312 | 0.231276 | 1.576212 | 1. 510977 | 0.114518 | 0. 30946 |
| C24 | 4.068609 | 0.083358 | 0. 202366 | 0.517413 | 0.573708 | 0. 10204 | 0.231902 |
| C25 | 4.322262 | 0.09 | 0.297221 | 1. 457857 | 2. 649429 | 0. 144606 | 0. 235975 |

**[Table2-2]**

| | | | | | | | |
|---|---|---|---|---|---|---|---|
| C26 | 4.515948 | 0.098495 | 0.246144 | 0.729312 | 0.522954 | 0.12011 | 0.321522 |
| C27 | 5.341681 | 0.093281 | 0.272263 | 0.800119 | 0.509819 | 0.142317 | 0.554986 |
| C28 | 3.407892 | 0.076799 | 0.232522 | 1.456796 | 1.97655 | 0.116423 | 0.228018 |
| C29 | 4.966603 | 0.113808 | 0.253573 | 0.820092 | 0.826504 | 0.140586 | 0.376179 |
| C30 | 4.387438 | 0.080235 | 0.205713 | 0.599781 | 0.448643 | 0.119357 | 0.388255 |
| C31 | 4.890419 | 0.091546 | 0.209689 | 0.490388 | 0.40576 | 0.130309 | 0.338561 |
| C32 | 4.743861 | 0.102853 | 0.210933 | 0.685307 | 0.46309 | 0.1275 | 0.371053 |
| C33 | 4.545896 | 0.078394 | 0.23639 | 0.706687 | 0.376478 | 0.128223 | 0.417527 |
| C34 | 4.879338 | 0.087853 | 0.24584 | 0.845795 | 0.802228 | 0.141971 | 0.354091 |
| C35 | 5.592855 | 0.143739 | 0.255895 | 0.59918 | 0.5216 | 0.156387 | 0.32664 |
| C36 | 3.168368 | 0.043542 | 0.156003 | 1.175999 | 0.821022 | 0.0948 | 0.30673 |
| C37 | 1.904159 | 0.03823 | 0.137382 | 0.913192 | 1.297416 | 0.059077 | 0.130488 |
| C38 | 5.133794 | 0.082412 | 0.250259 | 0.405698 | 0.306324 | 0.143895 | 0.307596 |
| C39 | 5.481245 | 0.100455 | 0.25799 | 1.21262 | 0.673192 | 0.153115 | 0.650402 |
| C40 | 5.066884 | 0.081058 | 0.206212 | 0.524986 | 0.374035 | 0.127949 | 0.46705 |
| C41 | 5.733472 | 0.105152 | 0.256386 | 0.812518 | 0.657904 | 0.156026 | 0.483332 |
| C42 | 3.174622 | 0.065422 | 0.192212 | 1.423082 | 1.330391 | 0.092209 | 0.263748 |
| C43 | 3.540378 | 0.08557 | 0.219788 | 0.724522 | 1.351046 | 0.101648 | 0.188128 |
| C44 | 4.867403 | 0.077836 | 0.211533 | 0.556858 | 0.341042 | 0.125475 | 0.404274 |
| C45 | 5.758942 | 0.139825 | 0.274267 | 0.661077 | 0.715905 | 0.143437 | 0.343581 |
| C46 | 5.12749 | 0.103567 | 0.241835 | 0.635045 | 0.6217 | 0.134999 | 0.363671 |
| C47 | 4.052743 | 0.088096 | 0.287635 | 1.530246 | 2.44845 | 0.142975 | 0.267871 |
| C48 | 3.538097 | 0.068013 | 0.207355 | 1.501998 | 1.46343 | 0.099127 | 0.271568 |
| C49 | 3.963864 | 0.068498 | 0.2028 | 0.783147 | 1.011967 | 0.113073 | 0.243063 |
| C50 | 4.674483 | 0.093736 | 0.234343 | 0.504228 | 0.601327 | 0.12437 | 0.29513 |

**[Table 2-3]**

| | | | | | | | |
|---|---|---|---|---|---|---|---|
| C51 | 5.10744 | 0.071939 | 0.260123 | 0.718617 | 0.487414 | 0.136544 | 0.383833 |
| C52 | 3.797152 | 0.08301 | 0.21197 | 1.257977 | 1.652317 | 0.121383 | 0.276324 |
| C53 | 3.666904 | 0.063608 | 0.212532 | 1.409882 | 1.397907 | 0.107528 | 0.27645 |
| C54 | 3.669759 | 0.060201 | 0.219733 | 1.160462 | 1.415053 | 0.119362 | 0.224945 |
| C55 | 5.577688 | 0.112842 | 0.297384 | 0.809138 | 0.944736 | 0.157022 | 0.40664 |
| C56 | 4.80013 | 0.112746 | 0.229434 | 0.484375 | 0.515209 | 0.130517 | 0.305638 |
| C57 | 3.996053 | 0.085922 | 0.226203 | 0.915829 | 1.325603 | 0.120302 | 0.218727 |
| C58 | 5.593568 | 0.106037 | 0.228564 | 0.922949 | 0.354298 | 0.137352 | 0.597271 |
| C59 | 4.183011 | 0.067032 | 0.215281 | 1.067118 | 0.510745 | 0.123266 | 0.563432 |
| C60 | 2.669424 | 0.043427 | 0.168425 | 1.377414 | 1.013359 | 0.094749 | 0.262207 |
| C61 | 3.933939 | 0.066949 | 0.187806 | 0.710891 | 0.430617 | 0.121007 | 0.397428 |
| C62 | 4.833593 | 0.109983 | 0.247826 | 1.007724 | 0.8055 | 0.158285 | 0.441962 |
| C63 | 3.82124 | 0.059047 | 0.183803 | 0.917524 | 0.423899 | 0.111269 | 0.388877 |
| C64 | 3.862166 | 0.078471 | 0.248281 | 1.103914 | 1.883512 | 0.136175 | 0.19022 |
| C65 | 4.246599 | 0.06329 | 0.198156 | 0.759043 | 0.394781 | 0.12433 | 0.424996 |
| C66 | 4.584282 | 0.068858 | 0.235684 | 0.827704 | 0.58921 | 0.139088 | 0.391183 |
| C67 | 2.873662 | 0.060177 | 0.187538 | 1.116132 | 1.351725 | 0.11568 | 0.225934 |
| C68 | 4.172895 | 0.068775 | 0.201496 | 0.955609 | 0.481429 | 0.135002 | 0.504855 |
| C69 | 3.372318 | 0.087526 | 0.249412 | 1.482401 | 1.949959 | 0.136829 | 0.226052 |
| C70 | 3.676624 | 0.084233 | 0.217029 | 1.020657 | 0.678275 | 0.133234 | 0.355831 |

**[Table 3-1]**

| Table 3: Genetic feature and steviol glycoside content in each individual in population C - (2) | | | | | | | |
|---|---|---|---|---|---|---|---|
| | RebN | RebE | RebG | TSG | Genetic feature | | |
| | | | | | (1) | (2) | (3) |
| C1 | 0.228456 | 0.27365 | 0.003213 | 8.702877 | - | - | - |
| C2 | 0.138086 | 0.039957 | 0.005633 | 6.298446 | + | + | + |
| C3 | 0.150392 | 0.039384 | 0.007065 | 7.609819 | + | - | + |
| C4 | 0.167489 | 0.176834 | 0.002733 | 6.957595 | - | - | - |
| C5 | 0.205748 | 0.195461 | 0.003001 | 7.701196 | - | - | + |
| C6 | 0.132129 | 0.025115 | 0.004761 | 6.507162 | + | + | + |
| C7 | 0.096153 | 0.01339 | 0.009211 | 6.313261 | + | + | + |
| C8 | 0.124732 | 0.017249 | 0.007279 | 6.926252 | - | + | + |
| C9 | 0.184852 | 0.204638 | 0.001756 | 6.38211 | + | + | + |
| C10 | 0.111264 | 0.029722 | 0.001124 | 3.770082 | - | - | - |
| C11 | 0.105507 | 0.017192 | 0.006355 | 4.755108 | + | + | + |
| C12 | 0.24091 | 0.275256 | 0.0022 | 8.034372 | - | - | - |
| C13 | 0.071695 | 0.011068 | 0.01103 | 4.797092 | + | + | + |
| C14 | 0.21104 | 0.278472 | 0.001721 | 6.963562 | - | - | - |
| C15 | 0.202025 | 0.058673 | 0.007464 | 9.224244 | + | - | + |
| C16 | 0.264633 | 0.332799 | 0.001698 | 7.240569 | - | - | - |
| C17 | 0.108421 | 0.026491 | 0.006455 | 5.062948 | + | + | + |
| C18 | 0.280548 | 0.188736 | 0.00212 | 6.87598 | - | - | - |
| C19 | 0.139254 | 0.024973 | 0.005035 | 5.466403 | + | + | + |
| C20 | 0.13661 | 0.025303 | 0.01139 | 8.133431 | - | + | + |
| C21 | 0.250121 | 0.189804 | 0.002614 | 7.597886 | + | + | + |
| C22 | 0.13745 | 0.021934 | 0.011681 | 7.056125 | + | + | + |
| C23 | 0.318027 | 0.268562 | 0.001995 | 7.667053 | + | + | + |
| C24 | 0.10826 | 0.023734 | 0.007658 | 5.919048 | + | + | + |
| C25 | 0.263485 | 0.273632 | 0.002991 | 9.737456 | - | - | - |

**[Table 3-2]**

| | | | | | | | |
|---|---|---|---|---|---|---|---|
| C26 | 0.144086 | 0.040809 | 0.006838 | 6.746219 | + | + | + |
| C27 | 0.195335 | 0.030094 | 0.00878 | 7.948674 | - | - | - |
| C28 | 0.266032 | 0.350049 | 0.001959 | 8.113041 | - | - | - |
| C29 | 0.146029 | 0.032115 | 0.00979 | 7.68528 | + | + | + |
| C30 | 0.123321 | 0.0187 | 0.008294 | 6.379737 | - | - | - |
| C31 | 0.079295 | 0.008707 | 0.015033 | 6.659706 | + | + | + |
| C32 | 0.094777 | 0.01785 | 0.008453 | 6.825676 | + | + | + |
| C33 | 0.160262 | 0.020189 | 0.007269 | 6.677315 | + | + | + |
| C34 | 0.146252 | 0.050114 | 0.007846 | 7.561328 | + | + | + |
| C35 | 0.098413 | 0.016337 | 0.018335 | 7.729382 | + | - | - |
| C36 | 0.195196 | 0.133655 | 0.00226 | 6.097576 | - | + | - |
| C37 | 0.175185 | 0.21796 | 0.0008 | 4.873889 | - | - | - |
| C38 | 0.10137 | 0.006095 | 0.011555 | 6.748997 | + | + | + |
| C39 | 0.227988 | 0.056694 | 0.007533 | 8.821234 | - | - | - |
| C40 | 0.106625 | 0.010004 | 0.013767 | 6.97857 | + | + | + |
| C41 | 0.139247 | 0.027188 | 0.013292 | 8.384517 | + | + | + |
| C42 | 0.275835 | 0.234386 | 0.001411 | 7.053319 | - | + | - |
| C43 | 0.157451 | 0.093047 | 0.00451 | 6.46609 | + | + | + |
| C44 | 0.114905 | 0.012597 | 0.010822 | 6.722744 | + | + | + |
| C45 | 0.130342 | 0.02425 | 0.013713 | 8.205338 | + | + | + |
| C46 | 0.114691 | 0.025839 | 0.010649 | 7.379486 | - | - | - |
| C47 | 0.289655 | 0.318794 | 0.002821 | 9.429286 | - | - | - |
| C48 | 0.255329 | 0.254696 | 0.002065 | 7.661676 | - | - | - |
| C49 | 0.176121 | 0.090631 | 0.004167 | 6.65733 | - | - | - |
| C50 | 0.108261 | 0.019991 | 0.009729 | 6.665599 | - | + | + |

**[Table 3-3]**

| | | | | | | | |
|---|---|---|---|---|---|---|---|
| C51 | 0. 173806 | 0. 026506 | 0.007625 | 7. 373848 | + | - | - |
| C52 | 0. 197111 | 0.172749 | 0. 002785 | 7. 772778 | - | - | - |
| C53 | 0.262506 | 0. 231025 | 0. 002202 | 7. 630546 | - | - | - |
| C54 | 0.206893 | 0. 206534 | 0. 002746 | 7. 285688 | - | - | - |
| C55 | 0.170517 | 0. 044699 | 0.01054 | 8. 531205 | + | + | + |
| C56 | 0. 109131 | 0.018136 | 0. 013236 | 6.718551 | + | + | + |
| C57 | 0. 156643 | 0. 122276 | 0. 003832 | 7.171391 | + | - | + |
| C58 | 0. 164649 | 0.01842 | 0.008692 | 8. 1318 | - | - | - |
| C59 | 0.200304 | 0. 068025 | 0. 005011 | 7. 003224 | - | - | - |
| C60 | 0. 220661 | 0.242012 | 0.001343 | 6. 093021 | - | - | - |
| C61 | 0. 114278 | 0.027849 | 0.006841 | 5. 997605 | + | + | + |
| C62 | 0. 145284 | 0. 04541 | 0.009551 | 7. 805119 | + | + | + |
| C63 | 0. 166812 | 0.041461 | 0.005605 | 6. 119538 | - | + | - |
| C64 | 0. 194819 | 0. 160685 | 0. 003623 | 7.861866 | + | + | + |
| C65 | 0. 179344 | 0.02184 | 0. 007307 | 6.419685 | - | - | - |
| C66 | 0. 146706 | 0.029441 | 0.008129 | 7. 020285 | - | + | + |
| C67 | 0.17041 | 0. 170815 | 0.002762 | 6. 274834 | - | - | - |
| C68 | 0. 136598 | 0. 034811 | 0.006346 | 6. 697816 | + | + | + |
| C69 | 0. 233747 | 0.266194 | 0.002587 | 8. 007026 | - | + | + |
| C70 | 0. 140362 | 0. 042173 | 0. 004972 | 6. 353391 | + | + | + |

From the above results, a tendency was found in which the content ratio of RebM to RebD in individual groups having the genetic feature(s) of the present invention was higher than that in individuals that did not have the genetic feature(s) of the present invention. In order to confirm whether the results were unique to the population C, the same study as above was made on other populations (populations D and E). The results are shown in Tables 4 to 7.

**[Table 4]**

| Table 4: Genetic feature and steviol glycoside content in each individual in population D - (1) | | | | | | | |
|---|---|---|---|---|---|---|---|
| | RebA | RebB | RebC | RebD | STV | RebF | RebM |
| D1 | 3.89314 | 0.056342 | 0.164304 | 0.56455 | 0.372864 | 0.121861 | 0.428764 |
| D2 | 1.429229 | 0.009044 | 0.332385 | 0.350715 | 8.326024 | 0.07054 | 0.031711 |
| D3 | 3.257867 | 0.034415 | 0.134642 | 0.708697 | 0.667679 | 0.086724 | 0.364196 |
| D4 | 4.16083 | 0.057925 | 0.175296 | 0.768336 | 0.655982 | 0.128367 | 0.42375 |
| D5 | 4.862509 | 0.053678 | 0.189697 | 0.618975 | 0.424163 | 0.123839 | 0.50201 |
| D6 | 4.866456 | 0.082855 | 0.25248 | 0.745 | 1.341955 | 0.143528 | 0.307506 |
| D7 | 4.191666 | 0.056862 | 0.168254 | 0.803681 | 0.505517 | 0.106343 | 0.430655 |
| D8 | 5.510896 | 0.070326 | 0.226055 | 0.60834 | 0.556508 | 0.149612 | 0.434224 |
| D9 | 4.56941 | 0.07079 | 0.214111 | 0.566472 | 0.592127 | 0.127374 | 0.407254 |
| D10 | 3.929294 | 0.043877 | 0.180003 | 0.790708 | 0.488609 | 0.109947 | 0.384154 |
| D11 | 5.268176 | 0.061944 | 0.253697 | 0.85194 | 0.631778 | 0.152267 | 0.56649 |
| D12 | 4.615484 | 0.057754 | 0.223051 | 0.538789 | 0.422727 | 0.140787 | 0.385013 |
| D13 | 4.464815 | 0.045141 | 0.17158 | 0.675008 | 0.296502 | 0.121954 | 0.501594 |
| D14 | 4.845151 | 0.056555 | 0.205676 | 0.640739 | 0.643056 | 0.138647 | 0.393458 |
| D15 | 4.785345 | 0.04606 | 0.213606 | 0.841651 | 0.519816 | 0.122997 | 0.487678 |
| D16 | 3.493794 | 0.060527 | 0.405769 | 1.640592 | 5.682108 | 0.136524 | 0.210585 |
| D17 | 3.500033 | 0.058823 | 0.187613 | 0.698962 | 0.674841 | 0.100846 | 0.2903 |
| D18 | 6.558848 | 0.075871 | 0.283718 | 0.882798 | 0.748198 | 0.170148 | 0.60458 |
| D19 | 5.883144 | 0.072871 | 0.202255 | 0.647267 | 0.538715 | 0.129045 | 0.563109 |

**[Table 5]**

| Table 5: Genetic feature and steviol glycoside content in each individual in population D - (2) | | | | | | | |
|---|---|---|---|---|---|---|---|
| | RebN | RebE | RebG | TSG | Genetic feature | | |
| | | | | | (1) | (2) | (3) |
| D1 | 0.195551 | 0.029.291 | 0.022924 | 5.849592 | + | + | + |
| D2 | 0.222554 | 0.407336 | 0.001212 | 11.18075 | + | - | - |
| D3 | 0.204749 | 0.062075 | 0.014429 | 5.535473 | + | - | - |
| D4 | 0.213538 | 0.067643 | 0.021041 | 6.672708 | - | - | - |
| D5 | 0.227532 | 0.029941 | 0.027726 | 7.060069 | + | - | + |
| D6 | 0.218615 | 0.097505 | 0.017094 | 8.072995 | - | - | - |
| D7 | 0.204151 | 0.059816 | 0.018816 | 6.545763 | - | - | - |
| D8 | 0.184117 | 0.038801 | 0.029304 | 7.808183 | + | + | + |
| D9 | 0.219927 | 0.036621 | 0.026802 | 6.830889 | - | + | - |
| D10 | 0.222371 | 0.074409 | 0.018621 | 6.241992 | - | - | - |
| D11 | 0.239466 | 0.041327 | 0.028073 | 8.095157 | + | + | + |
| D12 | 0.188742 | 0.024114 | 0.027363 | 6.623824 | + | + | + |
| D13 | 0.192024 | 0.02501 | 0.026856 | 6.520484 | + | + | + |
| D14 | 0.196012 | 0.041175 | 0.025163 | 7.185632 | - | - | - |
| D15 | 0.277432 | 0.072566 | 0.023764 | 7.390915 | - | - | - |
| D16 | 0.914819 | 0.805707 | 0.009424 | 13.35985 | - | + | - |
| D17 | 0.181951 | 0.090016 | 0.014099 | 5.797483 | - | + | - |
| D18 | 0.292307 | 0.049884 | 0.034963 | 9.701315 | + | + | + |
| D19 | 0.189892 | 0.027548 | 0.032505 | 8.28635 | + | + | + |

**[Table 6]**

| Table 6: Genetic feature and steviol glycoside content in each individual in population E - (1) | | | | | | | |
|---|---|---|---|---|---|---|---|
| | RebA | RebB | RebC | RebD | STV | RebF | RebM |
| E1 | 6.212014 | 0.08345 | 0.353489 | 1.527452 | 0.580656 | 0.179668 | 0.699396 |
| E2 | 5.494182 | 0.069253 | 0.293227 | 1.03409 | 0.336375 | 0.16077 | 0.525671 |
| E3 | 5.328516 | 0.081557 | 0.339177 | 1.370539 | 0.611505 | 0.155067 | 0.595811 |
| E4 | 5.062766 | 0.115883 | 0.273386 | 0.841787 | 0.558974 | 0.163897 | 0.497085 |
| E5 | 4.942109 | 0.114826 | 0.296842 | 1.543154 | 0.895254 | 0.167892 | 0.543025 |
| E6 | 4.804184 | 0.066037 | 0.308115 | 1.38401 | 0.633898 | 0.132978 | 0.580827 |
| E7 | 4.714854 | 0.100245 | 0.323253 | 1.258979 | 0.7609 | 0.168677 | 0.627197 |
| E8 | 4.536913 | 0.102653 | 0.333878 | 1.523125 | 1.172038 | 0.152576 | 0.496633 |
| E9 | 4.359091 | 0.058133 | 0.251408 | 1.215211 | 0.457543 | 0.131297 | 0.380999 |
| E10 | 4.320248 | 0.091127 | 0.333971 | 1.65393 | 1.266273 | 0.156107 | 0.431699 |
| E11 | 4.273264 | 0.077975 | 0.261763 | 0.957788 | 0.587064 | .136029 | 0.457316 |
| E12 | 4.192727 | 0.059748 | 0.25654 | 0.967966 | 0.412565 | 0.131087 | 0.56032 |
| E13 | 4.097659 | 0.070991 | 0.279959 | 1.267255 | 0.753104 | 0.133434 | 0.392471 |
| E14 | 4.047772 | 0.065048 | 0.264484 | 1.475195 | 0.653541 | 0.126641 | 0.37178 |
| E15 | 3.900042 | 0.056164 | 0.239808 | 1.203235 | 0.675194 | 0.11243 | 0.351592 |
| E16 | 3.757846 | 0.04953 | 0.235004 | 1.122951 | 0.566276 | 0.121198 | 0.38443 |
| E17 | 3.65434 | 0.058824 | 0.236257 | 0.802084 | 0.3828 | 0.106982 | 0.439652 |
| E18 | 2.958756 | 0.050949 | 0.193802 | 0.980468 | 0.664142 | 0.092961 | 0.23725 |
| E19 | 1.637071 | 0.021774 | 0.110002 | 0.580188 | 0.235393 | 0.045445 | 0.179728 |

**[Table 7]**

| Table 7: Genetic feature and steviol glycoside content in each individual in populationE - (2) | | | | | | | |
|---|---|---|---|---|---|---|---|
| | RebN | RebE | RebG | TSG | Genetic feature | | |
| | | | | | (1) | (2) | (3) |
| E1 | 0.435594 | 0.040229 | 0.028475 | 10.14042 | + | - | + |
| E2 | 0.247463 | 0.022732 | 0.031447 | 8.215209 | + | + | + |
| E3 | 0.327764 | 0.040688 | 0.025262 | 8.875887 | + | + | + |
| E4 | 0.245377 | 0.024755 | 0.031007 | 7.814917 | + | + | + |
| E5 | 0.298146 | 0.086386 | 0.018561 | 8.906195 | - | + | - |
| E6 | 0.364529 | 0.043525 | 0.021901 | 8.340002 | - | - | - |
| E7 | 0.307969 | 0.036533 | 0.020437 | 8.319044 | + | + | + |
| E8 | 0.305352 | 0.077788 | 0.014755 | 8.71571 | - | - | - |
| E9 | 0.243684 | 0.063472 | 0.019652 | 7.180491 | - | + | - |
| E10 | 0.319594 | 0.091746 | 0.012276 | 8.676971 | - | - | - |
| E11 | 0.282583 | 0.037514 | 0.02473 | 7.096026 | + | - | + |
| E12 | 0.260861 | 0.021816 | 0.023504 | 6.887134 | + | - | + |
| E13 | 0.261667 | 0.062925 | 0.014538 | 7.334003 | - | - | - |
| E14 | 0.320524 | 0.088621 | 0.012988 | 7.426595 | - | - | - |
| E15 | 0.230327 | 0.062189 | 0.015318 | 6.846298 | - | - | - |
| E16 | 0.23222 | 0.048419 | 0.013996 | 6.531871 | - | + | - |
| E17 | 0.207458 | 0.018175 | 0.020077 | 5.92665 | + | + | + |
| E18 | 0.18784 | 0.061801 | 0.010027 | 5.437996 | - | - | - |
| E19 | 0.123416 | 0.026538 | 0.006378 | 2.965934 | - | + | - |

The above results revealed that a similar tendency to above was also found in the populations D and E.

Next, the relationship between the content of each steviol glycoside measured and the genetic feature(s) of the present invention was studied. Specifically, statistically significant difference in the content of each steviol glycoside between individual groups having the genetic feature(s) of the present invention and the other individual groups in a combined population of the populations C to E was evaluated by the Welch t-test. The results are shown in Table 8. In the table, for example, results about "Genetic feature 1" are results of analyzing the content of each steviol glycoside in an individual group having the genetic feature (1) and the other individual groups (i.e., individual groups other than individuals having the genetic feature (1)); results about "Genetic feature 1+2" are results of analyzing the content of each steviol glycoside in an individual group having both the genetic features (1) and (2) and the other individual groups (i.e., individual groups other than individuals having both the genetic features (1) and (2)); and results about "Genetic feature 1/2/3" are results of analyzing the content of each steviol glycoside in an individual group having at least one of the genetic features (1), (2) and (3) and the other individual groups (i.e., individual groups that did not have any of the genetic features (1), (2) and (3)). "+" and "++" represent that the individual groups having the genetic feature(s) had a larger content than that in individual groups of the other individuals with P < 0.05 and P < 0.01, respectively; "-" and "--" represent that the individual groups having the genetic feature(s) had a smaller content than that in individual groups of the other individuals with P < 0.05 and P < 0.01, respectively; and "±" represents that no significant difference at a significance level of 0.05 was found between the contents in both the individual groups.

**[Table 8]**

| Table 8: Relationship between genetic feature and steviol glycoside content | | | | | | | | |
|---|---|---|---|---|---|---|---|---|
| | Genetic feature | | | | | | | |
| | 1 | 2 | 3 | 1+2 | 2+3 | 1+3 | 1+2+3 | 1/2/3 |
| RebA | ++ | ± | ++ | ++ | ++ | ++ | ++ | + |
| RebB | + | ± | ++ | + | ++ | ++ | + | + |
| RebC | ± | ± | ± | ± | ± | ± | ± | ± |
| RebD | -- | - | -- | -- | -- | -- | -- | -- |
| RebE | -- | - | -- | -- | -- | -- | -- | -- |
| RebF | + | ± | ++ | ± | + | ++ | | ± |
| RebG | ++ | ± | + | + | ± | ++ | ± ± | ++ |
| RebM | + | ± | + | ± | ± | ++ | ± | ± |
| RebN | -- | - | -- | -- | -- | -- | -- | - |
| STV | ± | - | -- | -- | -- | -- | -- | ± |
| TSG | ± | ± | ± | ± | ± | ± | ± | ± |

The above results revealed that an individual having the genetic feature(s) of the present invention has tendencies in which the content of RebA, RebB, RebF, RebG and/or RebM is large and/or tendencies in which the content of RebD, RebE, RebN and/or stevioside is small as compared with an individual that does not have the genetic feature(s) of the present invention. The results also revealed that the above tendencies can be most detected with a lower significance level by focusing on a combination of the genetic features (1) and (3). The number of tendencies that can be detected was the genetic feature (3) and a combination of the genetic features (1) and (3) (the number of tendencies: 9), the genetic feature (1) (the number of tendencies: 8), a combination of the genetic features (1) and (2), a combination of the genetic features (2) and (3), and a combination of the genetic features (1) to (3) (the number of tendencies: 7), at least one of the genetic features (1) to (3) (the number of tendencies: 6), and the genetic feature (2) (the number of tendencies: 4) in descending order.

Those skilled in the art shall understand that a large number of various changes or modifications can be made without departing from the spirit of the present invention. Thus, it should be understood that the embodiments of the present invention described herein are mere illustrations and do not intend to limit the scope of the present invention.

[Sequence Listing]

## Claims

1. A method of screening for a stevia plant having at least one of the following chemical features (a) to (r), comprising a step of detecting from the genome of a test stevia plant the presence and/or the absence of at least one of the following genetic features (1) to (3).
(1) Heterozygous or homozygous for the allele wherein the base at the position corresponding to position 184 of SEQ ID NO: 1 is G.
(2) Heterozygous or homozygous for the allele wherein the base at the position corresponding to position 170 of SEQ ID NO: 2 is C.
(3) Heterozygous or homozygous for the allele wherein the base at the position corresponding to position 92 of SEQ ID NO: 3 is C.
(a) The content ratio of rebaudioside (Reb) M to RebD is high as compared with a stevia plant that does not have any of the genetic features (1) to (3).
(b) The content ratio of RebB to RebE is high as compared with a stevia plant that does not have any of the genetic features (1) to (3).
(c) The content ratio of RebG to RebE is high as compared with a stevia plant that does not have any of the genetic features (1) to (3).
(d) The content ratio of RebG to stevioside is high as compared with a stevia plant that does not have any of the genetic features (1) to (3).
(e) The content of RebA is large as compared with a stevia plant that does not have any of the genetic features (1) to (3).
(f) The content of RebB is large as compared with a stevia plant that does not have any of the genetic features (1) to (3).
(g) The content of RebF is large as compared with a stevia plant that does not have any of the genetic features (1) to (3).
(h) The content of RebG is large as compared with a stevia plant that does not have any of the genetic features (1) to (3).
(i) The content of RebM is large as compared with a stevia plant that does not have any of the genetic features (1) to (3).
(j) The content of a steviol glycoside having a β-1,3-glucoside bond is large as compared with a stevia plant that does not have any of the genetic features (1) to (3).
(k) The content of RebD is small as compared with a stevia plant that does not have any of the genetic features (1) to (3).
(l) The content of RebE is small as compared with a stevia plant that does not have any of the genetic features (1) to (3).
(m) The content of RebN is small as compared with a stevia plant that does not have any of the genetic features (1) to (3).
(n) The content of stevioside is small as compared with a stevia plant that does not have any of the genetic features (1) to (3).
(o) The content of a steviol glycoside having no β-1,3-glucoside bond is small as compared with a stevia plant that does not have any of the genetic features (1) to (3).
(p) The content ratio of any one of RebA, RebB, RebF, RebG, and RebM to any one of RebD, RebE, RebN, and stevioside is high as compared with a stevia plant that does not have any of the genetic features (1) to (3).
(q) The content ratio of a steviol glycoside having a β-1,3-glucoside bond to a steviol glycoside having no β-1,3-glucoside bond is high as compared with a stevia plant that does not have any of the genetic features (1) to (3).
(r) The content ratio of a steviol glycoside produced by UGT76G1 from its substrate steviol glycoside to the substrate steviol glycoside of UGT76G1 is high as compared with a stevia plant that does not have any of the genetic features (1) to (3).

2. The method according to claim 1, further comprising a step of measuring the content of RebA, RebB, RebD, RebE, RebF, RebG, RebM, RebN and/or stevioside in a test stevia plant tissue in which the presence and/or the absence of the genetic features has/have been detected.

3. The method according to claim 1 or 2, wherein the step of detecting the presence and/or the absence of the genetic features is performed by use of CAPS method, dCAPS method or TaqMan PCR method.

4. A screening kit for a stevia plant having at least one of the chemical features (a) to (r) defined in claim 1, comprising a reagent for detecting the presence and/or the absence of at least one of the genetic features (1) to (3) defined in claim 1.

5. The kit according to claim 4, wherein the reagent comprises a primer and/or a probe for use in CAPS method, dCAPS method or TaqMan PCR method.

6. A stevia plant having at least one of the genetic features (1) to (3) defined in claim 1.

7. The plant according to claim 6, wherein the plant has at least one of the chemical features (a) to (r) defined in claim 1.

8. The plant according to claim 6 or 7, wherein the plant is a non-genetically modified plant.

9. The plant according to any one of claims 6 to 8, wherein the plant includes a stevia plant subjected to a mutagenesis treatment and a progeny plant thereof.

10. A seed, a tissue, a dried leaf, a tissue culture or a cell of the plant according to any one of claims 6 to 9.

11. The tissue, tissue culture or cell according to claim 10, which is selected from an embryo, a meristem cell, a pollen, a leaf, a root, a root apex, a petal, a protoplast, a leaf section and a callus.

12. A method of producing a stevia plant having at least one of the chemical features (a) to (r) defined in claim 1, the method comprising a step of crossing the stevia plant according to any one of claims 6 to 9 with a second stevia plant.

13. The method according to claim 12, wherein the second plant is the stevia plant according to any one of claims 6 to 9.

14. A method of producing a stevia plant having at least one of the chemical features (a) to (r) defined in claim 1, comprising a step of modifying the genome of a stevia plant such that the genome has at least one of the genetic features (1) to (3) defined in claim 1.

15. The method according to claim 14, wherein the modification of the genome is performed by a mutagenesis treatment.

16. An extract of the plant according to any one of claims 6 to 9, or of the seed, tissue, dried leaf, tissue culture or cell according to claim 10 or 11, wherein the extract has at least one of the following chemical features (a') to (r').
(a') The content ratio of rebaudioside (Reb) M to RebD is high as compared with an extract of a stevia plant that does not have any of the genetic features (1) to (3) defined in claim 1.
(b') The content ratio of RebB to RebE is high as compared with an extract of a stevia plant that does not have any of the genetic features (1) to (3) defined in claim 1.
(c') The content ratio of RebG to RebE is high as compared with an extract of a stevia plant that does not have any of the genetic features (1) to (3) defined in claim 1.
(d') The content ratio of RebG to stevioside is high as compared with an extract of a stevia plant that does not have any of the genetic features (1) to (3) defined in claim 1.
(e') The content of RebA is large as compared with an extract of a stevia plant that does not have any of the genetic features (1) to (3) defined in claim 1.
(f') The content of RebB is large as compared with an extract of a stevia plant that does not have any of the genetic features (1) to (3) defined in claim 1.
(g') The content of RebF is large as compared with an extract of a stevia plant that does not have any of the genetic features (1) to (3) defined in claim 1.
(h') The content of RebG is large as compared with an extract of a stevia plant that does not have any of the genetic features (1) to (3) defined in claim 1.
(i') The content of RebM is large as compared with an extract of a stevia plant that does not have any of the genetic features (1) to (3) defined in claim 1.
(j') The content of a steviol glycoside having a β-1,3-glucoside bond is large as compared with an extract of a stevia plant that does not have any of the genetic features (1) to (3) defined in claim 1.
(k') The content of RebD is small as compared with an extract of a stevia plant that does not have any of the genetic features (1) to (3) defined in claim 1.
(l') The content of RebE is small as compared with an extract of a stevia plant that does not have any of the genetic features (1) to (3) defined in claim 1.
(m') The content of RebN is small as compared with an extract of a stevia plant that does not have any of the genetic features (1) to (3) defined in claim 1.
(n') The content of stevioside is small as compared with an extract of a stevia plant that does not have any of the genetic features (1) to (3) defined in claim 1.
(o') The content of a steviol glycoside having no β-1,3-glucoside bond is small as compared with an extract of a stevia plant that does not have any of the genetic features (1) to (3) defined in claim 1.
(p') The content ratio of any one of RebA, RebB, RebF, RebG, and RebM to any one of RebD, RebE, RebN, and stevioside is high as compared with an extract of a stevia plant that does not have any of the genetic features (1) to (3) defined in claim 1.
(q') The content ratio of a steviol glycoside having a β-1,3-glucoside bond to a steviol glycoside having no β-1,3-glucoside bond is high as compared with an extract of a stevia plant that does not have any of the genetic features (1) to (3) defined in claim 1.
(r') The content ratio of a steviol glycoside produced by UGT76G1 from its substrate steviol glycoside to the substrate steviol glycoside of UGT76G1 is high as compared with an extract of a stevia plant that does not have any of the genetic features (1) to (3) defined in claim 1.

17. A method of producing an extract having at least one of the chemical features (a') to (r') defined in claim 16, comprising a step of obtaining an extract from the plant according to any one of claims 6 to 9, or from the seed, tissue, dried leaf, tissue culture or cell according to claim 10 or 11.

18. A method of producing a steviol glycoside having a β-1,3-glucoside bond, comprising a step of purifying the steviol glycoside having a β-1,3-glucoside bond from the extract according to claim 16.

19. A method of producing a food, a sweetener composition, a flavor or a medicament, comprising:
a step of providing an extract of the plant according to any one of claims 6 to 9, an extract of the seed, tissue, dried leaf, tissue culture or cell according to claim 10 or 11, or the extract according to claim 16; and
a step of adding the extract to a raw material for the food, sweetener composition, flavor or medicament.
